# EUROPÄISCHE PATENTSCHRIFT

(11) **EP 2 751 565 B1**
(45) Veröffentlichungstag und Bekanntmachung des Hinweises auf die Patenterteilung: **05.10.2022**
(21) Anmeldenummer: 12772707.1
(22) Anmeldetag: 31.08.2012
(51) Int. Cl.: G01N 27/327, B01L 3/00, G01N 33/543

(54) **TRÄGERMATERIAL FÜR ELEKTRISCH POLARISIERBARE BIOMATERIALIEN, POLYELEKTROLYTMATERIALIEN, ATOME, IONEN UND MOLEKÜLE; DESSEN HERSTELLUNG UND VERWENDUNG**
CARRIER MATERIAL FOR ELECTRICALLY POLARISABLE BIOMATERIALS, POLYELECTROLYTE MATERIALS, ATOMS, IONS AND MOLECULES; PRODUCTION AND USE THEREOF
MATÉRIAU SUPPORT POUR BIOMATÉRIAUX, MATÉRIAUX POLYÉLECTROLYTIQUES, ATOMES. IONS ET MOLÉCULES ÉLECTRIQUEMENT POLARISABLES, LEUR PRODUCTION ET LEUR UTILISATION

(30) Priorität: 31.08.2011 DE 102011053174; 07.11.2011 DE 102011055115; 16.07.2012 DE 102012106365
(43) Veröffentlichungstag der Anmeldung: 09.07.2014
(73) Patentinhaber: Helmholtz-Zentrum Dresden - Rossendorf e.V., 01328 Dresden (DE); Leibniz-Institut für Polymerforschung Dresden e.V., 01069 Dresden (DE)
(72) Erfinder: SCHMIDT, Heidemarie, 01187 Dresden (DE); BAUMGART, Christine, 01069 Dresden (DE); SKORUPA, Ilona, 01108 Dresden (DE); SCHMIDT, Oliver, G., 01328 Dresden (DE); MÜLLER, Martin, 01328 Dresden (DE); HELM, Manfred, 01277 Dresden (DE)
(74) Vertreter: Kailuweit & Uhlemann Patentanwälte Partnerschaft mbB
(86) Internationale Anmeldenummer: PCT/DE2012/200058
(87) Internationale Veröffentlichungsnummer: WO 2013/029609

(56) Entgegenhaltungen:
- EP-A1- 1 843 157
- EP-A1- 2 198 953
- EP-B1- 0 645 811
- WO-A1-00/73777
- WO-A1-98/01758
- DE-A1- 2 055 269
- DE-A1- 2 055 269
- DE-A1- 10 344 915
- US-A1- 2002 149 019
- US-A1- 2002 149 019
- US-A1- 2003 211 637
- US-A1- 2004 011 650
- US-B1- 6 538 298
- US-B1- 6 538 298
- LEE C ET AL: "Sol-gel derived PNNZT thin films for micromachined piezoelectric force sensors", THIN SOLID FILMS, ELSEVIER-SEQUOIA S.A. LAUSANNE, CH, Bd. 299, Nr. 1-2, 15. Mai 1997 (1997-05-15), Seiten 88-93, XP004115476, ISSN: 0040-6090, DOI: 10.1016/S0040-6090(96)09453-9
- SCHMIDT HEIDEMARIE ET AL: "Kelvin probe force microscopy for characterizing doped semiconductors for future sensor applications in nano- and biotechnology", APPLIED SURFACE SCIENCE, Bd. 281, 2013, Seiten 24-29, XP028679139, ISSN: 0169-4332, DOI: 10.1016/J.APSUSC.2013.04.080
- NESTEROV ALEXANDER ET AL: "Precise selective deposition of microparticles on electrodes of microelectronic chips", REVIEW OF SCIENTIFIC INSTRUMENTS, AIP, MELVILLE, NY, US, Bd. 79, Nr. 3, 24. März 2008 (2008-03-24), Seiten 35106-35106, XP012115311, ISSN: 0034-6748, DOI: 10.1063/1.2900012
- K. KÖNIG ET AL: "Programmable high voltage CMOS chips for particle-based high-density combinatorial peptide synthesis", SENSORS AND ACTUATORS B: CHEMICAL, Bd. 147, Nr. 2, 3. Juni 2010 (2010-06-03), Seiten 418-427, XP055046698, ISSN: 0925-4005, DOI: 10.1016/j.snb.2009.12.039
- CHIOU PEI YU ET AL: "Massively parallel manipulation of single cells and microparticles using optical images", NATURE: INTERNATIONAL WEEKLY JOURNAL OF SCIENCE, NATURE PUBLISHING GROUP, UNITED KINGDOM, Bd. 436, 21. Juli 2005 (2005-07-21), Seiten 370-372, XP002367223, ISSN: 0028-0836, DOI: 10.1038/NATURE03831
- RONALD PETHIG: "Dielectrophoresis: Status of the theory, technology, and applications", BIOMICROFLUIDICS, Bd. 4, Nr. 2, 1. Januar 2010 (2010-01-01), Seite 022811, XP055046612, ISSN: 1932-1058, DOI: 10.1063/1.3456626
- VOLDMAN JOEL: "Electrical forces for microscale cell manipulation", ANNUAL REVIEW OF BIOMEDICAL ENGINEERING, Bd. 8, 2006, Seiten 425-454, XP002688869, ISSN: 1523-9829
- WANG X-B AND CHENG J ED - CHENG ET AL: "Electronic manipulation of cells on microchip-based devices", 1. Januar 2001 (2001-01-01), BIOCHIP TECHNOLOGY, AMSTERDAM : HARWOOD ACADEMIC PUBL, PAGE(S) 135 - 139, XP009165496, ISBN: 90-5702-613-9 das ganze Dokument
- MANARESI N ET AL: "A CMOS chip for individual cell manipulation and detection", SOLID-STATE CIRCUITS CONFERENCE, 2003. DIGEST OF TECHNICAL PAPERS. ISS CC. 2003 IEEE INTERNATIONAL SAN FRANCISCO, CA, USA 9-13 FEB. 2003, PISCATAWAY, NJ, USA,IEEE, US, 9. Februar 2003 (2003-02-09), Seiten 1-10, XP010661609, ISBN: 978-0-7803-7707-3
- FUCHS A B ET AL: "Electronic sorting and recovery of single live cells from microlite sized samples", LAB ON A CHIP, ROYAL SOCIETY OF CHEMISTRY, Bd. 6, 1. Januar 2006 (2006-01-01), Seiten 121-126, XP008108127, ISSN: 1473-0197, DOI: 10.1039/B505884H [gefunden am 2005-11-15]
- GASTROCK G ET AL: "Sampling and monitoring in bioprocessing using microtechniques.", JOURNAL OF BIOTECHNOLOGY DEC 2001, Bd. 82, Nr. 2, Dezember 2001 (2001-12), Seiten 123-135, XP002693854, ISSN: 0168-1656
- LEE C ET AL: "Sol-gel derived PNNZT thin films for micromachined piezoelectric force sensors", THIN SOLID FILMS, ELSEVIER-SEQUOIA S.A. LAUSANNE, CH, vol. 299, no. 1-2, 15 May 1997 (1997-05-15), pages 88-93, XP004115476, ISSN: 0040-6090, DOI: 10.1016/S0040-6090(96)09453-9
- SCHMIDT HEIDEMARIE ET AL: "Kelvin probe force microscopy for characterizing doped semiconductors for future sensor applications in nano- and biotechnology", APPLIED SURFACE SCIENCE, vol. 281, 2013, pages 24-29, XP028679139, ISSN: 0169-4332, DOI: 10.1016/J.APSUSC.2013.04.080

## Beschreibung

Die Erfindung betrifft Trägermaterialien (Carrier) für elektrisch polarisierbare Biomaterialien, für strukturierte einkomponentige Polyelektrolytschichten, mehrkomponentige Polyelektrolytmultischichten oder Schichten vorgebildeter Polyelektrolytkomplexe, für Atome, Ionen und/oder Moleküle **(epAIMP),** die Herstellung bzw. die Ausführungsvarianten dieser Trägermaterialien zur Manipulation, Modifikation und Bewegung einzelner **epAIMP** bis zur Konstruktion molekularer Maschinen **mM.**

Unter Manipulation von **epAIMP** wird im Sinne der Erfindung das Anziehen (Binden), das Abstoßen und das Sortieren von **epAIMP** an das Trägermaterial verstanden.

Unter Modifikation von **epAIMP** wird im Sinne der Erfindung das Verändern von einzelnen chemischen und/oder physikalischen Eigenschaften der **epAIMP** am Trägermaterial verstanden.

Unter Bewegung von **epAIMP** wird im Sinne der Erfindung die gezielt gerichtete Fortbewegung der **epAIMP** auf oder nahe der Oberfläche des Trägermaterials verstanden.

### Stand der Technik

Elektrisch polarisierbare Biomaterialien, die strukturierten, einkomponentigen Polyelektrolytschichten, die mehrkomponentigen Polyelektrolytmultischichten oder die Schichten vorgebildeter Polyelektrolytkomplexe, die Atome, Ionen und/oder Moleküle (epAIMP), richten sich in einem elektrischen Feld aus. In einem homogenen elektrischen Feld wirkt auf den elektrischen Dipol der **epAIMP** ein Drehmoment, welches den Dipol in Richtung des elektrischen Feldes ausrichtet. Im inhomogenen elektrischen Feld erfährt der Dipol der **epAIMP** eine Kraft, die ihn in Gebiete höherer Feldstärke hineinzieht.

Die Dynamik der wirkenden Kräfte bestimmt, ob die Trägheitskräfte, welche der Bewegung von **epAIMP** in einem homogenen oder inhomogenen elektrischen Feld entgegenwirken, überwunden werden können. Eine empfohlene Lösung ist die Stick- und Slip-Methode, d.h. das Wirken einer langsam bewegenden Kraft in die gewünschte Richtung und ein schnelles Abstellen der bewegenden Kraft.

Ein Biomaterial ist definiert als Substanz natürlichen oder künstlichen Ursprungs, die zeitweilig oder dauerhaft, eigenständig oder als Teil eines Ganzen, jedwedes Gewebe, Organ oder jegliche Funktion des Körpers behandelt, verbessert oder ersetzt (D. F. Williams: Definitions in Biomaterials. Progress in biomedical Engineering, 4 ed. Elsevier, Amsterdam 1987.).

Biomoleküle sind Moleküle organischer Substanzen, die in Lebewesen vorkommen. Biomoleküle stehen mit Biomaterialien in Wechselwirkung und definieren die biologische Umgebung für Biomaterialien. Im Sinne der Erfindung werden unter Biomoleküle alle natürlichen und künstliche Materialien verstanden,
∘ die in Lebewesen vorkommen oder von Lebewesen gebildet werden,
∘ die künstlich hergestellt werden und natürliche, organische Substanzen nachempfinden, oder
∘ an die sich andere (künstliche) Substanzen oder Metalle anlagern können oder angelagert haben und/oder die mit anderen künstlichen oder natürlichen Materialien Verbindungen eingegangen sind.

Biomoleküle können elektrostatische Ladungen tragen und damit einen elektrischen Dipol ausbilden. Solche Biomoleküle zählen zu den epAIMP. Die Dipol-Dipol-Wechselwirkungen der polaren Biomoleküle sorgen für die Ausrichtung der Biomoleküle untereinander. Auf der Nanometer-Längenskala gehört die elektrostatische Wechselwirkung zu den Kräften mit der größten Stärke und auch zu denen mit der größten Reichweite. Elektrostatische Kräfte sind auch sehr stark in wässrigen Lösungen und halten so die Biomoleküle voneinander fern.

Bei der Untersuchung von Biomaterialien oder Biomolekülen muss das Trägermaterial die Biomaterialien räumlich und zeitlich begrenzbar aufnehmen und die Biomaterialien für lokale Anforderungen zur Verfügung stellen. Dabei spielt die Zelladhäsion (Zell-Haftfähigkeit) von Biomaterialien und Biomolekülen an dem Trägermaterial sowie die Zellbeweglichkeit eine wichtige Rolle. Die Mechanismen, die der Zelladhäsion oder Zellbeweglichkeit zu Grunde liegen, sind nicht vollständig erforscht.

Er werden biologisch abbaubare und biologisch nicht abbaubare Trägermaterialien für Biomaterialien unterschieden.

Biomoleküle können hydrophob oder hydrophil sein. Hydrophobe Biomoleküle sind unpolar. Hydrophile Biomoleküle sind polar und entweder positiv (basisch) oder negativ (sauer) aufladbar.

Das invertierte optische oder konfokale Mikroskop ermöglicht die Quantifizierung der einzelnen Zell-Zell- und Zell-Oberflächen-Wechselwirkungen unter physiologischen Bedingungen. Eine Vielzahl von wichtigen Parametern der zellulären Adhäsion, wie zum Beispiel die maximale Zelladhäsionskraft, einzelne Entfaltungsereignisse, die Tether-Charakteristik sowie die Gesamtenergie der elektrostatischen Bindung können damit bestimmt werden.

Um Einzelmoleküluntersuchungen mittels Fluoreszenzdetektion an Biomolekülen unter nahezu physiologischen Bedingungen durchführen zu können, müssen die Biomoleküle eine hohe Fluoreszenzaktivität besitzen. Da die wenigsten Moleküle intrinsisch fluoreszierend sind bzw. ihre Fluoreszenzeigenschaften den Anforderungen nicht entsprechen, werden biologisch funktionale Moleküle in der Regel über die kovalente Bindung von eigens zu diesem Zweck entwickelten Farbstoffen markiert (Sauer, Han et al. 1993).

Biomoleküle mit ungepaarten Elektronen weisen ein magnetisches Moment auf, das etwa drei Größenordnungen stärker als das Moment eines Protons ist. Mit Methoden der Elektronen-Spin-Resonanz-Spektroskopie (EPR) kann dieses Moment in hochempfindlichen Messungen als Sonde dienen, um strukturelle Informationen auf der atomaren bis hin zur Nanometerskala zu gewinnen.

Um nun Biosensoren zu konstruieren, müssen die biologisch aktiven Elemente an Trägerstoffen fixiert werden. Zur Fixierung gibt es die unterschiedlichsten Methoden, man unterscheidet physikalische und chemische Methoden. Zu den physikalischen Methoden zählt vorrangig die Adsorption. Sie ist die einfachste Methode. Auf diesem Wege hergestellte Biosensoren reagieren aufgrund der reversiblen Natur des Bindungsgleichgewichts allerdings auf Änderungen der Umgebungsbedingungen empfindlich. Zu den chemischen Methoden zählen die kovalente Kopplung und die Vernetzung. Bei der kovalenten Kopplung an derivatisierte, wasserunlösliche Träger dürfen nur diejenigen Gruppen des Biomoleküls beteiligt sein, die nicht für die biologische Aktivität zuständig sind.

WO/2005/007387 A beschreibt die Nanomanipulation des piezoelektrischen Trägermaterials durch das Anlegen einer elektrischen Spannung, was zu einer Änderung der verspannten Kontaktfläche zwischen Trägermaterial und Biomaterial bzw. Biomolekül führt. Der Nachteil ist, dass diese piezoelektrischen Trägermaterialien dünn sein müssen, damit die anzulegenden elektrischen Spannungen nicht zu groß werden.

Trotz der enormen technologischen Fortschritte in den vergangenen Jahren gibt es nur wenige biosensorische Ansätze, mit denen sich ein Pharma-Screening realisieren lässt. Gründe hierfür sind, dass der "klassische" Biosensor auf der Grundlage von Enzymen und Antikörpern zumeist nur strukturelle und keine funktionellen Informationen liefert und die biomolekulare Interaktionsanalyse mit Biosensoren sehr anspruchsvoll ist. Jedoch können durch Mikro- und Nanotechnologien dem Pharma-Screening mittels Biosensoren neue Impulse verliehen werden. Insbesondere erscheint das "High Content Screening" (HCS), ein paralleles Screening von vielen Effekten, mit Biosensoren realisierbar zu sein und könnte einen Ausweg aus den sich derzeit anbahnenden Engpässen in den Entwicklungs-Pipelines vieler Pharmaunternehmen darstellen.

Biosensoren wurden in den vergangenen Jahren für vielfältige analytische Aufgaben entwickelt. Dabei standen Anwendungen aus dem klinisch-medizinischen Bereich, der Fermentationskontrolle, der Qualitätskontrolle von Lebensmitteln und der Umweltanalytik im Vordergrund. Jedoch sind die Einsatzmöglichkeiten von biosensorischen Techniken weitaus vielfältiger: Mit Biosensoren lassen sich einerseits sehr spezifisch Strukturelemente erkennen, die für potentielle Arzneistoffe charakteristisch sind; andererseits können Assays realisiert werden, mit denen sich dann Arzneistoffwirkungen erfassen lassen. Ein Biosensor besteht typischerweise aus einem biologischen Erkennungselement und einem physikalischen Sensor (Transducer). Biosensoren werden meist nach dem Funktionsprinzip ihres Signalwandlers eingeteilt. Man unterscheidet im Wesentlichen elektrochemische, optische, kalorimetrische und mikrogravimetrische Biosensoren. Bei komplexen Systemen, die ganze lebende Zellen enthalten, ist eine solche Einteilung jedoch nicht sinnvoll.

Die Polyelektrolytmaterialien besitzen lokale elektrische Dipole, das heißt wahlweise positiv oder negativ geladene funktionelle Gruppen und können durch attraktive elektrostatische Wechselwirkung mit den entgegengesetzt geladen wirkenden Kräften oder Ladungszentren der Trägermaterialien gebunden werden (Physisorption, Adsorption). Solche Polyelektrolytmaterialien zählen zu den epAIMP.

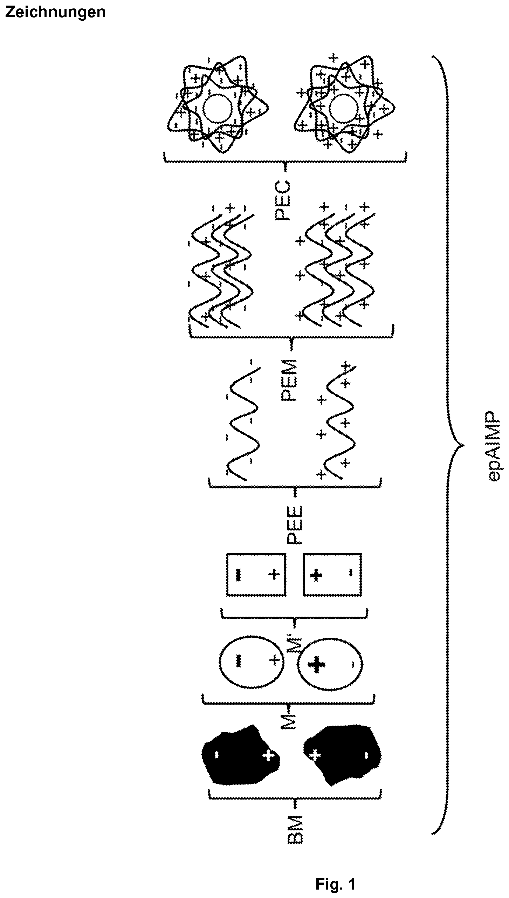

Die Polyelektrolytmaterialien (PEL) [M. Schmidt (Vol. Ed.): Polyelectrolytes with Defined Molecular Architecture I, Springer, 2004.] umfassen einkomponentige Polyelektrolytsysteme (PEE), deren Ladungen nur ein Vorzeichen besitzen oder welche nur einen Polyelektrolyttypen (z.B. negativ geladene Polyacrylsäure) enthalten, sowie Polyelektrolyt-Mischsysteme, wie z.B. Polyelektrolytmultischichten (PEM) oder vorgebildete Polyelektrolytkomplexpartikel (PEC). Die verschiedenen Polyelektrolytmaterialien sind in Fig. 1 skizziert.

Einkomponentige PEL-Systeme (PEE) werden durch einfache Adsorption aus Lösungen des jeweiligen PEL in geeigneter Konzentration an den Trägermaterialien gebildet. Bei herkömmlichen Substraten (geringere Oberflächenladungsdichte als die hier beschriebenen) bilden sich hierbei meist nur inhomogene PEL-Schichten (Inseln) aufgrund der elektrostatischen Selbstrepulsion zwischen Oberflächenregionen mit bereits adsorbierten PEL-Schichten und restlichen PEL in Lösung. Die hier anvisierten Trägermaterialien besitzen wesentlich höhere Oberflächenladungsdichten (Werte s. unten). Dadurch können einkomponentige PEL-Schichten mit wesentlich höherem, über eine Monolage hinausgehendem Belegungsgrad entstehen oder zu adsorbierten PEL-Mengen führen, die deutlich von denen abweichen, die bisher experimentell an hochgeladenen Substraten gemessen wurden. Möglicherweise können dadurch neue Erkenntnisse zur Theorie der Polyelektrolytadsorption an festen Oberflächen [z.B. Fleer et al., Polymers at Interfaces, Chapman, 1993, R.R. Netz, J.F. Joanny, Macromolecules, 32, 9013 (1999), A.V. Dobrynin, M. Rubinstein, Prog. Polym. Sci. 30, 1049-1118 (2005)] gewonnen werden oder diese besser in Einklang mit experimentellen Befunden zur Abhängigkeit der adsorbierten PEL-Menge von der Oberflächenladungsdichte gebracht werden.

PEM werden dagegen durch konsekutive Adsorption von Polykationen (PEL mit positiv geladenen funktionellen Gruppen oder Monomereinheiten) mit Polyanionen (PEL mit negativ geladenen Gruppen oder Monomereinheiten) an ein Substrat hergestellt, wobei das Trägermaterial beispielsweise ein Siliziumträger ist, dessen Oberfläche chemisch und/oder physikalisch behandelt wird [WO 2010 066432 A]. In diesem Fall ist die Ursache für die Adsorption und Desorption chemisch und physikalisch nicht von der Umgebung isoliert.

Polyelektrolytkomplexpartikel (PEC) werden zunächst direkt durch Mischen einer Polykation- und Polyanionlösung im nichtstöchiometrischen Verhältnis in der Volumenphase gebildet [US 2008/0058229 A1]. Die sich dabei je nach Mischungsverhältnis bildenden Partikel mit neutralem Kern und positiv oder negativ geladener Schale können ähnlich den PEL durch Physisorption an das geladene Trägermaterial binden.

PEM-Filme können durch Verwendung kettensteifer PEL an z.B. unidirektional texturierten Trägern orientierte Nanostrukturen bilden [M. Müller, Orientation of a-helical Poly(L-lysine) in Consecutively Adsorbed Polyelectrolyte Multilayers on Texturized Silicon Substrates, Biomacromolecules, 2(1), 262-269 (2001)].

Ebenso können durch Verwendung kettensteifer PEL [ M. Müller, T. Reihs, W. Ouyang, Needle like and spherical polyelectrolyte complex nanoparticles of poly(L-lysine) and copolymers of maleic acid, Langmuir, 21(1), 465-469 (2005)] stäbchenförmige PEC-Partikel gebildet werden, welche an unidirektional texturierten Trägern auch orientierte Nanostrukturen erzeugen können.

Orientierte PEM-Filme oder PEC-Filme können das Zellwachstum beeinflussen und zu einem Ersatz von plasmamodifizierten Substraten führen, welche mit wesentlich höherem Aufwand hergestellt werden.

Als einkomponentige PEL-Materialien oder in PEM oder PEC können auch leitfähige PEL und Polymere wie z.B. Polyanilin, Polypyrrol, Polythiophen oder Polyethylendioxythiophen (PEDOT) einbezogen und inkorporiert werden und als leitende Haftvermittler zwischen Indiumzinnoxid (ITO)- und aktiven Farbstoffschichten für organische lichtemittierende Dioden (OLEDs) verwendet werden.

Diese Polyelektrolytmaterialien können aufgrund ihrer strukturellen Verwandtschaft oder auch Identität zu Biomaterialien (Proteine, Polysaccharide, Polynucleotide) wiederum als Haftmaterial für andere Materialien, z.B. Biomoleküle und Biomaterialien, dienen. Dabei können kontrolliert inerte Passivierungsschichten (PEL-1) oder aktiv bindende Schichten (PEL-2) oder auch biozide Schichten (z.B. Bakterien) (PEL-3) für Biomaterialien, Biofluide oder Zellen entstehen.

Eine aktuelle Fragestellung ist die Bestimmung der Geschwindigkeit, der Stärke und/oder der Spezifität der Bindung, sowie die Bestimmung der Konzentration von aktiven Biomolekülen und Partikeln sowie die Identifizierung von neuen Wechselwirkungspartnern ("Ligandenfishing") an Polyelektrolytmaterialien. Biomoleküle können hydrophob oder hydrophil sein. Hydrophobe Biomoleküle sind unpolar. Hydrophile Biomoleküle sind polar und können im Kontakt zum Lösungsmittel entweder positiv geladene (basische), negativ geladene (saure) Ladungszentren (funktionelle Gruppen) besitzen oder elektrostatisch neutral sein.. Einige niedrigmolekulare Biomoleküle wie Aminosäuren, Monosaccharide und Nukleotide stellen reaktive Monomere für die Polymerisation zu hochmolekularen Biomolekülen, den Biopolymeren wie Proteinen (z.B. Kollagen, Serumalbumin, Insulin), Polysacchariden (z.B. Glykogen, Stärke, Cellulose, Dextrane, Chitin) und Polynukleotiden (z.B. DNA, RNA) dar.

Fig. 1 zeigt verschiedene bereits elektrisch polarisierte oder elektrisch polarisierbare Biomaterialien **(BM),** Atome, Ionen und/oder Moleküle) vor **(M_{A}, M_{B}, M_{C})** und nach der Modifizierung **(M_{A}', M_{B}', M_{C}'),** Einkomponentige Polyelektrolytsysteme **(PEE),** Polyelektrolytmultischichten **(PEM)** und Polyelektrolytkomplexartikel **(PEC).** In den Abbildungen wird nicht zwischen polarisierbaren Biomaterialien (BM), Atomen, Ionen oder Molekülen und Einkomponentigen Polyelektrolytsystemen **(PEE),** Polyelektrolytmultischichten **(PEM)** und Polyelektrolytkomplexartikel **(PEC)** unterschieden. Fig. 1 zeigt im unteren Teil der Abbildung den Aufbau der Polyelektrolytmaterialien (PEL) mit einer positiven Überschussladung und im oberen Teil den Aufbau der PEL mit einer negativen Überschussladung. Diese werden in den weiteren Abbildungen durch ein Oval mit einem "+" bzw. "-" entsprechend ihrer Überschussladung dargestellt. Diese elektrisch polarisierbaren Teilchen werden mit **epAIMP** bezeichnet. Die **epAIMP,** welche schematisch in verschiedenen Größen und Formen gezeichnet sind, unterscheiden sich bezüglich ihrer elektrischen Polarisierbarkeit, ihrer Masse und ihrer Tendenz, sich mit anderen Biomaterialien, Atomen, Ionen und/oder Molekülen oder Polyelektrolytmaterialien zu verbinden. Das erfindungsgemäße Trägermaterial nutzt die elektrische Polarisierbarkeit der **epAIMP,** welche ein Maß für die Verschiebbarkeit von positiver relativ zu negativer Ladung ist. Dabei können Elektronenwolken relativ zum positiven schweren Kern in Atomen und Ionen und/oder positive Ionen relativ zu negativen Ionen in Molekülen verschoben werden. Es können auch komplexere **epAIMP** mit höheren elektrischen Multipolen auftreten und miteinander elektrostatisch wechselwirken. Zur Vereinfachung werden nur elektrische Dipole in den Zeichnungen dargestellt.

Die **epAIMP** können einen permanenten elektrischen Dipol aufweisen und/oder in elektrischen Feldern einen elektrischen Dipol ausbilden. Die Dipol-Dipol-Wechselwirkungen von **epAIMP** sorgen für deren Ausrichtung untereinander. Auf der Nanometer-Längenskala gehört die elektrostatische Wechselwirkung zu den Wechselwirkungen mit der größten Kraft und mit der größten Reichweite. Elektrostatische Kräfte sind auch sehr stark in wässrigen Lösungen und halten so **epAIMP** voneinander fern. Die **epAIMP** kann nicht nur als Dipol, sondern auch als komplexere **epAIMP** mit höheren elektrischen Multipolen auftreten und miteinander elektrostatisch wechselwirken. Zur Vereinfachung werden nur elektrische Dipole in den Zeichnungen dargestellt.

Die Bewegung der **epAIMP** gelingt in elektrischen Feldern zwischen zwei Elektroden, an die eine elektrische Spannung von außen angelegt wird.

Einzelne bewegliche Ionen können in einem Festkörper mit Rückseitenelektrode und mit statischer oder positionierbarer Vorderseitenelektrode, z.B. metallisch leitende Messspitze eines Atomaren Kraftmikroskopes, bewegt werden. [N. Balke u.a.: Nanoscale mapping of ion diffusion in a lithium-ion battery cathode. NATURE NANOTECHNOLOGY 5 (2010) 749-754].

Außerhalb eines Festkörpers können **epAIMP** durch Dielektrophorese [Pei Yu Chiou u.a.: Massively parallel manipulation of single cells and microparticles using optical images. NATURE 436 (2005) 370-372] zwischen der Oberfläche eines Trägermaterials und einer großflächigen Vorderseitenelektrode, welche in einem Abstand von der Oberfläche des Trägermaterials angebracht ist und dieses nicht berührt, bewegt werden. Dazu wird die Rückseite des Trägermaterials durch eine Schattenmaske beleuchtet. Die photogenerierten Ladungsträger im Carrier erhöhen lokal die Leitfähigkeit des Carriers und verschieben lokal die Position der Rückseitenelektrode von der unteren ITO-Schicht in das undotierte a-Si:H des Carriers. Dadurch wird lokal der Abstand zwischen der oberen ITO-Schicht (Vorderseitenelektrode) mit unveränderlicher Position und der Rückseitenelektrode mit veränderlicher Position verändert. Die zwischen der Vorderseitenelektrode und der Rückseitenelektrode abfallende Spannung verursacht ein lokal variierendes elektrisches Feld. Die Richtung des elektrischen Feldes wird durch die Polarität der an der Vorder- und Rückseitenelektrode angelegten Spannung bestimmt. Es lassen sich sehr schnell durch Wechseln der Schattenmaske unterschiedliche Positionen der Rückseitenelektrode auf der µm-Skale einstellen.

Eine molekulare Maschine ist aus einer diskreten Zahl von epAIMP konstruiert und erfüllt spezielle Funktionen auf der Nanometer- und/oder Mikrometer-Längenskale. Ihre Arbeitsweise ist in vielerlei Hinsicht ähnlich den von Menschen geschaffenen Maschinen. Es ist ein Mechanismus, bei dem Einzelteile ineinanderpassen, sich bewegen und zusammenspielen, um eine bestimmte Aufgabe zu erfüllen [Goodsell, David S : Wirtschaft und Produktion in der molekularen Welt, Übersetzt von Hummel, Isolde, 2. Aufl., 2010. Spektrum Akademischer Verlag.].

Molekulare Maschinen produzieren eine mechanische Bewegung (Output) als Antwort auf eine spezifische Anregung (Input). Es gibt künstliche und biologische molekulare Maschinen.

Im erweiterten Sinn werden alle Moleküle, welche Funktionen von der Meter-Längenskale auf die Nanometer- und/oder Mikrometer-Längenskale übertragen und dort ausführen, als molekulare Maschinen bezeichnet.

Die **epAIMP** zum Aufbau der molekularen Maschinen gibt es nur in wenigen Formen und Größen. Zum Beispiel können künstliche molekulare Maschinen aus Rotaxanen und Catenanen aufgebaut sein. Biologische molekulare Maschinen sind weitgehend aus sechs Arten von Atomen, Kohlenstoff, Sauerstoff, Stickstoff, Schwefel, Phosphor und Wasserstoff, sowie weitgehend aus vier Arten von Molekülen, Proteinen, Nucleinsäuren, Lipiden und Polysacchariden aufgebaut.

Wenn Atome, Ionen und/oder Moleküle aufeinandertreffen, treten sie miteinander in Wechselwirkung. Ist diese Wechselwirkung schwach, z.B. in langsamen Neutralgasen, dann setzen die Atome und/oder Moleküle nach dem Zusammenstoß ihren Weg unverändert fort. Ist die Wechselwirkung stark, z.B. in Gasen mit Ionen oder in schnellen Neutralgasen, dann setzen die Atome, Ionen und/oder Moleküle nach dem Stoß, bei dem Coulombkräfte zwischen Ionen und resonanter Ladungstransfer zwischen neutralen Atomen und Molekülen wirken können, ihren Weg verändert fort.

Die diskrete Zahl von atomaren, ionischen und/oder molekularen Komponenten wird nach einem Bauplan mittels der Grundkonzepte - chemische Komplementarität und Hydrophobizität - zu künstlichen und biologischen molekularen Maschinen zusammengesetzt, die jeweils optimal auf eine bestimmte Rolle zugeschnitten sind.

Ist die Wechselwirkung komplementär, z.B. wenn Atom - und Molekülbereiche perfekt zu einem ähnlich geformten Atom - und Molekülbereich eines Nachbarmoleküls passen, entsteht eine feste Verbindung.

Molekulare Maschinen nutzen zwei spezielle Bindungsarten: Wasserstoffbrücken zwischen einem Wasserstoffatom und einem Sauerstoff- oder Stickstoffatom sowie Salzbrücken zwischen Atomen, Ionen, und/oder Molekülen, die eine entgegengesetzte elektrische Ladung tragen. Diese speziellen Bindungen funktionieren wie kleine Klammern, die Atome, Ionen und Moleküle aneinanderkoppeln.

Die Ausbildung einer speziellen Bindungsart kann durch eine Modifikation der **epAIMP** katalysiert werden.

Eine großflächige Modifikation von wärmeempfindlichen **epAIMP** gelingt durch Wärmezufuhr. Die Wärmeenergie kann z.B. durch Ummagnetisierung von magnetisierbaren Teilchen in einem von außen angelegten Magnetfeld [US 2008 0319247 A1], durch Absorption von elektromagnetischen Wellen und/oder durch Anregung von Gitterschwingungen durch 10 Nanosekunden lange Laserpulse mit einer Energiedichte von 20 W/mm² erzeugt werden [M. E. Msall u.a.: Ballistic phonon production in photoexcited Ge, GaAs, and Si. PHYS. REV. B. 65 (2002) 195205-1-7].

Zu den bereits synthetisierten einfachen künstlichen molekularen Maschinen zählen Motoren [Javier Vicario u.a.: Controlling the speed of rotation in molecular motors. Dramatic acceleration of the rotary motion by structural modification, CHEM. COMMUN. (2005) 5910-5912], Propeller [B. Wang u. a.: Chemically Tunable Nanoscale Propellers of Liquids. PHYS. REV. LETT. 98 (2007) 266102-1-4], Schalter [Jean-Pierre Desvergne u.a.: Cation complexing photochromic materials involving bisanthracenes linked by a polyether chain. Preparation of a crown-ether by photocycloisomerization; J. CHEM. SOC., CHEM. COMMUN. (1978) 403-404], Molekültransporter, Molekülzangen, Molekülsensoren und logische Elemente [Jonathan E. Green u.a.: A 160-kilobit molecular electronic memory patterned at 1011 bits per square centimeter. NATURE 445 (2007) 414-417].

Die Herstellung molekularer Maschinen ist eine wesentliche Voraussetzung für aktuelle Forschungen auf dem Gebiet des Molekülassemblings, der DNA-Maschinen, der nanoelektromechanischen Systeme, der Nanosensoren und der Protein-Dynamik.

Komplexe molekulare Maschinen wurden bereits theoretisch konzipiert, konnten jedoch bisher nicht getestet werden, da bisher keine Verfahren zur Fertigung dieser komplexen molekularen Maschinen existieren.

Molekulare Sensoren interagieren mit einem Analyt und zeigen eine messbare Änderung an. Häufig werden Fluoreszenzen in Etherresten an den molekularen Sensoren zur Messung der Änderung nach Interaktion verwendet.

### Zu lösende Nachteile

Die bisher entwickelten Trägermaterialien sind nicht geeignet, um einzelne **epAIMP** räumlich und zeitlich begrenzt aufzunehmen, zu modifizieren und zu bewegen. Mit den bisher bekannten Trägermaterialien lassen sich keine molekularen Maschinen aus den elektrisch polarisierbaren Atomen, Ionen, Molekülen, Biomaterialien oder Polyelektrolymaterialien herstellen.

Bei der Elektrophorese [Pei Yu Chiou u.a.: Massively parallel manipulation of single cells and microparticles using optical images. NATURE. 436 (2005) 370-372.] ist die Richtung des elektrischen Feldes zu einem gegebenen Zeitpunkt überall gleich. Bei zu langer Beleuchtung der Rückseitenelektrode diffundieren die photogenerierten Ladungsträger auch in das undotierte a-Si:H hinein, erhöhen die Leitfähigkeit des undotierten a-Si:H überall und verschieben damit großflächig die Position der Rückseitenelektrode von der unteren ITO-Schicht in das undotierte a-Si:H. Außerdem ist das Anlegen einer elektrischen Spannung zwischen der Vorderseitenelektrode, welche sich in einem Abstand von der Oberfläche des Trägermaterials (Nitridschicht) befindet, und der Rückseitenelektrode nachteilig, da der Abstand zwischen der Nitridschicht und der Vorderseitenelektrode größer sein muss als die Partikel oder Zellen, damit sich diese mechanisch frei auf der Nitridschicht bewegen können.

Bei der Elektrophorese ist die Ausbreitung des elektrischen Feldes zwischen der Vorderseitenelektrode und der Rückseitenelektrode nicht homogen, da die Partikel und/oder Zellen aufgrund ihrer dielektrischen Eigenschaften die elektrischen Feldlinien anders weiterleiten als das umgebende Material. Damit beeinflussen die Partikel und/oder Zellen das elektrische Feld, welches eigentlich zum Beeinflussen der Partikel und/oder Zellen verwendet werden soll.

Bei der Elektrophorese können kleinere Partikel und/oder Zellen (<1 Mikrometer) nicht sortiert werden, da die Lokalisierung der Rückseitenelektrode durch die Diffusion der photogenerierten Ladungsträger limitiert ist und da das elektrische Feld zwischen der Vorderseitenelektrode und der Rückseitenelektrode nicht groß genug ist, um die Brownsche Bewegung der Partikel und/oder Zellen zu überwinden.

Bei der Elektrophorese kann die Detektion der lokalisierten Partikel und/oder Zellen nur optisch durch die Vorderseitenelektrode hindurch erfolgen. Nimmt man die Vorderseitenelektrode weg, dann bildet sich kein elektrisches Feld aus und die Partikel und Zellen können nicht lokalisiert werden. Eine Detektion der lokalisierten Partikel mittels Atomarer Kraftmikroskop-Messungen oder mittels Raster-Elektronenmikroskopie-Messungen ist durch die Vorderseitenelektrode hindurch nicht möglich.

### Aufgabe der Erfindung

Die Aufgabe der Erfindung ist es, ein Trägermaterial für die Manipulation, Modifikation und Bewegung von elektrisch polarisierbaren Biomaterialien, Atomen, Ionen, Molekülen oder Polyelektrolytmaterialien anzugeben. Das Trägermaterial soll eine lokal steuerbare Adsorption und Desorption der Materialien gewährleisten.

Das Polyelektrolytmaterial kann aus einkomponentigen Polyelektrolytsystemen **(PEE),** aus mehrkomponentigen Polyelektrolytmultischichten **(PEM)** -oder aus vorgebildeten Polyelektrolytkomplexpartikeln **(PEC)** bestehen. Die Herstellung und die möglichen Ausführungsvarianten des Trägermaterials, z.B. zur lokalen Manipulation und Modifikation und zum translatorischen und/oder rotatorischen Transport einzelner **epAIMP** sowie die Verwendung des Trägermaterials zur räumlich und zeitlich begrenzbaren Aufnahme der **epAIMP** zur Fertigung molekularer Maschinen **mM,** sollen beschrieben werden. Dabei spielt die Adhäsion (Haftfähigkeit) von **epAIMP** an dem Trägermaterial sowie die Beweglichkeit der **epAIMP** eine wichtige Rolle. Die Adhäsion und der Beweglichkeit von **epAIMP** soll vom Trägermaterial kontrolliert werden.

### Grundzüge des Lösungsweges

Ausgangspunkt für das Trägermaterial ist ein lokal dotiertes Halbleitermaterial oder ein piezoelektrisches Material. Auf Oberfläche kann optional eine isolierende Deckschicht aufgebracht sein. Das Trägermaterial stellt eine Quelle für lokal steuerbare, oberflächennahe elektrostatische Kräfte dar, die die epAIMPs manipulieren, modifizieren und/oder bewegen können. Im Gegensatz zum bisherigen Stand der Technik werden diese oberflächennahen elektrostatischen Kräfte nicht von der Umgebung beeinflusst, die oberflächennahen, elektrostatischen Kräfte sind chemisch von der Umgebung isoliert.

Die **epAIMP** können sich durch unterschiedlich starke oberflächennahe, elektrostatische Kräfte an oder bis zu wenige Nanometern über der Oberfläche eines Trägermaterials oder an anderen **epAIMP,** die nahe oder an der Oberfläche des Trägermaterials angelagert sind, ausrichten.

Die hohe Oberflächenladungsdichte der Trägermaterialien führt generell zu einer höheren adsorbierten Menge der epAIMP, z. B. von einkomponentigen PEL-Materialien, von PEM- und PEC-Systemen im Vergleich zu sonstigen Substraten (z.B. Silizium-Wafer). Durch die Wahl der Spezies (Elektronen oder Löcher) und/oder der Konzentration der Majoritätsladungsträger im lokal dotierten Halbleiter oder durch die Bildung von Domänen im piezoelektrischen Material wird die Richtung und Stärke der oberflächennahen, elektrostatischen Kräfte an der Oberfläche des Trägermaterials variiert. Durch Dotierung werden Volumenladungsdichten von 10¹⁵ bis 10²¹ e/cm³ und Flächenladungsdichten von 10¹⁰ bis 10¹⁴ e/cm² im Halbleiter erreicht. Die Flächenladungsdichte in piezo- und ferroelektrischen Materialien beträgt 10¹³ bis 10¹⁵ e/cm².

Die Dicke der isolierenden Deckschicht und/oder die lokalen Ladungen in der isolierenden Deckschicht bestimmen die Stärke der oberflächennahen, elektrostatischen Kräfte. Die Reichweite der elektrostatischen Kräfte beträgt bis zu 100 nm oberhalb der Oberfläche des Trägermaterials. Damit kann über die Dicke dieser Deckschicht die adsorbierte Menge der epAIMP kontrolliert eingestellt werden.

Die Strukturierung der isolierenden Deckschicht und/oder des darunterliegenden Halbleiters bzw. piezoelektrischen Materials bestimmt die Richtung und das Ausmaß der oberflächennahen, elektrostatischen Kräfte. Damit können **epAIMP-**Materialien strukturiert oder lokal kontrolliert abgeschieden werden

Strukturierte einkomponentige und PEL-, mehrkomponentige PEM- und PEC-Schichten, Biomaterialien Ionen, Atome und Moleküle **(epAIMP)** werden durch Änderung der Richtung und Stärke oberflächennaher, elektrostatischer Kräfte durch Anlegen einer Spannung an der Rückseitenelektrode des Trägermaterials manipuliert, das heißt adsorbiert oder desorbiert.

Übergangsbereiche mit jeweils umgekehrter Richtung der oberflächennahen, elektrostatischen Kräfte können im Trägermaterial gezielt ausgebildet werden und durch Anlegen einer Spannung an eine oder mehrere Rückseitenelektroden des Trägermaterials zum unterschiedlichen Manipulieren der epAIMP verwendet werden.

Durch Nutzung der Ausbildung intrinsischer elektrischer Felder in den Raumladungszonen (Übergangsbereiche im Halbleiter) von lokalen Dotierprofilen kann durch Anlegen einer Spannung an die Rückseitenelektrode ein noch stärkeres Manipulieren der oberflächennahen, elektrostatischen Kräfte erfolgen. Damit kann auch die Adsorption der epAIMP gesteuert werden.

Die Erzeugung von Domänen mit umgekehrter Richtung der oberflächennahen, elektrostatischen Kräfte im piezoelektrischen Material (Übergangsbereiche im piezoelektrischen Material) erfolgt durch lokale Strukturierung des piezoelektrischen Materials.

Als isolierende Schicht, als Halbleitermaterial oder als piezoelektrisches Material kann ein optisch aktives Material und/oder als geladene Störstelle im Trägermaterial kann ein magnetisierbares Material zur spezifischen lokalen Anregung oder Adsorption von epAIMP verwendet werden.

Durch Strukturierung der Rückseitenelektrode sowie der implantierten Bereiche kann eine Crossbar-Struktur zum lokalen Sortieren und Manipulieren von epAIMP erreicht werden.

Das Trägermaterial kann aus einzelnen Trägermaterialzellen des Arrays lückenlos und überlappungsfrei durch verschiedene Polygone gebildet werden und jede oder einzelne Trägermaterialzellen sind mit einer eigenen metallisch leitenden Rückseitenelektrode ausgestattet. Im Folgenden wird zur einfacheren Beschreibung und Verwendung der erfindungsgemäßen Arraystruktur eine viereckige Polygonstruktur angenommen.

Durch Nutzung der Ausbildung von elektrischen Gradientenfeldern im oberflächennahen Bereich des Trägermaterials können die **epAIMP** durch Anlegen einer Spannung an die Rückseitenelektrode noch stärker mittels oberflächennaher, elektrostatischer Kräfte manipuliert werden.

Die Ausbildung starker elektrischer Gradientenfelder kann an der Grenze zwischen p-type Halbleiter p und n-type Halbleiter n oder an der Grenze zwischen Domänen mit entgegengesetzt gerichtetem intrinsischen elektrischen Feld erfolgen.

Die Erzeugung von p-type Halbleiter p und n-type Halbleiter n kann mittels Ionenimplantation und anschließender thermischer Ausheilung erfolgen.

Übergangsbereiche zwischen benachbarten Trägermaterialzellen mit jeweils entgegengesetzter Richtung der oberflächennahen, elektrostatischen Kräfte können im Trägermaterial gezielt ausgebildet werden und durch Anlegen einer Spannung an eine oder mehrere Rückseitenelektroden des Trägermaterials zum Transport einzelner **epAIMP** innerhalb einzelner und zwischen benachbarten Trägermaterialzellen verwendet werden.

Durch das Einbringen von magnetisierbaren Nano- oder Mikroteilchen in die isolierende Schicht oder in den oberflächennahen Bereich einzelner Trägermaterialzellen, können an diesen Trägermaterialzellen lokalisierte, temperaturempfindliche **epAIMP** durch Anlegen eines äußeren Magnetfeldes und der Wärmeentwicklung bei der Ummagnetisierung der magnetischen Teilchen modifiziert werden.

Zur lokalen Anregung von Gitterschwingungen kann das Trägermaterial mit unterschiedlichen Elektron-Phonon-Wechselwirkungseigenschaften der einzelnen Trägermaterialzellen großflächig beleuchtet werden. Aufgrund der Elektronen-Phonon-Wechselwirkung ist die Umwandlung von Lichtenergie in Gitterschwingungsenergie in den Trägermaterialzellen mit einer großen Elektron-Phonon-Wechselwirkung am größten.

Molekulare Maschinen können durch den sequentiellen Transport von **epAIMP** an die Trägermaterialzellen, an denen die molekularen Maschinen gebildet werden, hergestellt werden. Optional können einzelne **epAIMP** vor/nach/während des Transports modifiziert werden. Diese Modifikation kann zur Katalyse der Verbindung zwischen epAIM, aus denen die molekularen Maschinen gebildet werden, realisiert werden.

### Erzeugte Vorteile oder Verbesserungen gegenüber dem Stand der Technik

Ein Hauptvorteil ist die Kompatibilität des Trägermaterials mit der Mikroelektronik und die Verwendung einer chemisch resistenten und biokompatiblen isolierenden Deckschicht sowie die Wiederverwendbarkeit des Trägermaterials. Ein weiterer Vorteil ist die zeitlich und/oder räumlich veränderliche Manipulation, Modifizierung und der Transport einzelner **epAIM.** Der Bereich des Trägermaterials, von dem die oberflächennahen elektrostatischen Kräfte aufgrund der Umverteilung von Ladungen im oberflächennahen Bereich des Trägermaterials ausgehen, ist räumlich und chemisch vollständig vom adsorbierten **epAIMP** getrennt. Das heißt, es wirken nur die oberflächennahen, elektrostatischen Kräfte, es findet aber kein direkter Kontakt zu den Ladungen im oberflächennahen Bereich des Trägermaterials statt. Die **epAIM,** aus denen molekulare Maschinen **mM** zusammengesetzt sind, können durch gezielte Anziehung an das Trägermaterial und spätere Abstoßung vom Trägermaterial aus Gasen, Flüssigkeiten und Pulvern gefiltert werden. Zur Sortierung von **epAIMP** mit unterschiedlicher Masse und unterschiedlicher elektrischer Polarisierbarkeit kann der Slip-Stick-Mechanismus unter Ausnutzung der Dynamik der Bewegung genutzt werden.

Ein weiterer Vorteil ist die Verfügbarkeit der epAIMP, speziell der PEL-Materialien, und die Anwendung einfacher nasschemischer Prozesse mit Wasser als Lösungsmittel. Andere Lösungsmittel können auch verwendet werden.

Ein weiterer Vorteil ist die stufenlos regelbare Oberflächenladungsdichte, wenn das Trägermaterial ein Halbleiter ist, und die stufenweise regelbare Oberflächenladungsdichte, wenn das Trägermaterial ein piezo- oder ferroelektrisches Material ist. Da die Adsorption von **epAIMP,** zum Beispiel von PEL-Materialien, direkt von der Ladungsdichte abhängig ist, kann damit eine kontrollierte Abscheidung von **epAIMP** erzielt werden.

Ein weiterer Vorteil ist die zeitlich und/oder räumlich veränderliche Adsorption und Desorption, d. h. man kann mit dem durch die **epAIMP** modifizierten Trägermaterialien Komponenten aus Flüssigkeiten oder Gemischen filtern durch gezielte Anziehung (Adsorption) an das Trägermaterial und spätere Abstoßung (Desorption) vom Trägermaterial.

Ein weiterer Vorteil ist die ausschließliche Strukturierbarkeit der oberflächennahen Bereiche des Trägermaterials durch elektrostatischen Kräfte und Oberflächenladung hinsichtlich deren Vorzeichen und Amplitude, ohne dass andere Oberflächeneigenschaften wie Rauhigkeit, Morphologie, Benetzung, Konzentration und Zusammensetzung funktioneller Gruppen geändert werden. Auf das **epAIMP** wirken immer nur die kurzreichweitigen van der Waals-Kräfte der isolierenden Deckschicht und die langreichweitigen, oberflächennahen, elektrostatischen Kräfte der Oberflächenladung. Das hat Vorteile, da bei der mengenmäßig und lokal kontrollierten Abscheidung von **epAIMP** nur ein Parameter, die Oberflächenladung, berücksichtigt werden muss.

Im Besonderen können über die Trägermaterialien unidirektional orientierte Ladungsmuster erzeugt werden und diese als Templat für die selektive Adsorption und Ausrichtung von **epAIMP,** zum Beispiel von PEL-Materialien aus kettensteifen PEL, verwendet werden. Vorteile gegenüber mechanischen oder auf mechanischer Streckung beruhender Texturierungsverfahren werden erwartet.

Solche nach Absatz [0081] gezielt unidirektional und nach Absatz [0082] gezielt und musterkontrolliert mit **epAIMP** modifizierte und strukturierte Oberflächen der Trägermaterialien können zu einer unidirektional gerichteten oder selektiven Wechselwirkung oder einem ebensolchen Wachstum von Zellen führen.

Auf Grund der chemischen Isolierung der Ursache der elektrostatischen Kräfte von der Umgebung werden eventuelle chemische Reaktionen und damit die Beeinflussung der elektrostatischen Kräfte vermieden.

### Bildbeschreibung

Die Ausführungsbeispiele werden mit Abbildungen beschrieben.

Fig. 2 zeigt ein Trägermaterial **TM** aus halbleitenden (a) und aus piezo- oder ferroelektrischem (b) Material mit oberflächennaher elektrostatischer Kraft **(4)** (a) mit unterschiedliche Stärke und (b) mit gleicher Stärke.
Fig. 3 zeigt ein Trägermaterial **TM** aus halbleitendem (a) und aus piezo- oder ferroelektrischem (b) Material mit strukturierter isolierender Deckschicht (1) mit oberflächennaher elektrostatischer Kraft **(4)** mit unterschiedlicher Stärke.
Fig. 4 zeigt ein Trägermaterial **TM** aus halbleitenden (a) und aus piezo- oder ferroelektrischen (b) Material mit isolierender Deckschicht **(1)** mit geladenen Störstellen in der isolierenden Deckschicht (1).
Fig. 5 zeigt ein Trägermaterial **TM** aus strukturiertem, halbleitenden (a) und strukturiertem, piezoelektrischen (b) Material mit isolierender Deckschicht (1) mit oberflächennaher elektrostatischer Kraft **(4)** mit verschiedener Richtung und gleicher Stärke.
Fig. 6 zeigt ein Trägermaterial **TM** aus halbleitendem Material mit isolierender Deckschicht (1) mit metallisch leitender Rückseitenelektrode (5) und minimierter oberflächennaher elektrostatischer Kraft **(4)** durch Anlegen einer Gleichspannung (Kelvin-Spannung).
Fig. 7 zeigt ein Trägermaterial **TM** mit großflächiger, metallisch leitender Rückseitenelektrode **(5)** aus halbleitendem Material (a) mit Löchern **(p⁺)** und mit Elektronen **(n⁺)** und mit der Grenze **(7)** zwischen n-Halbleiter und p-Halbleiter und aus piezo- oder ferroelektrischem Material (b) mit Domänen mit entgegengesetzt gerichteten intrinsischen elektrischen Feldern und mit der Grenze zwischen Domänen mit entgegengesetzt gerichteten intrinsischen elektrischen Feldern **(8b).**
Fig. 8 zeigt ein Trägermaterial **TM** aus halbleitendem Material (a) mit Löchern **(p)** und mit Elektronen **(n)** und mit der Grenze **(7)** zwischen n-Halbleiter und p-Halbleiter und aus piezoelektrischem Material (b) mit Domänen mit entgegengesetzt gerichteten intrinsischen elektrischen Feldern und mit der Grenze zwischen Domänen mit entgegengesetzt gerichteten intrinsischen elektrischen Feldern **(8b)** mit strukturierter metallisch leitender Rückseitenelektrode **(5).**
Fig. 9 zeigt die lückenlose und überlappungsfreie Einteilung des Trägermaterials in gleichförmige Trägermaterialzellen **TMZ(i,j).**
Fig. 10 zeigt den Schnitt **XY** durch vier Trägermaterialzellen eines halbleitenden Trägermaterials mit strukturierter Bottom-Elektrode für Translationsbewegungen von **epAIMP.**
Fig. 11 zeigt schematisch die Translation eines **epAIMP** aufgrund der oberflächennahen, elektrostatischen Kräfte.
Fig. 12 zeigt den Schnitt **X'Y'** durch vier Trägermaterialzellen eines halbleitenden Trägermaterials mit strukturierter Rückseiten-Elektrode für Rotationsbewegungen.
Fig. 13 zeigt schematisch die Rotation eines **epAIMP** aufgrund der oberflächennahen, elektrostatischen Kräfte.
Fig. 14 zeigt die sukzessive Zusammensetzung einer molekularen Maschine aus den Komponenten **epAIMP_{A}, epAIMP_{B}** und **epAIMP_{C}.**
Fig. 15 zeigt die durch elektromagnetische Wellen **22** modifizierten, elektrisch polarisierbaren Biomaterialien, Polyelektrolytmaterialien, Atomen, Ionen und/oder Moleküle **(epAIMP_{A}')** in der **TMZ(1,1)** und in der **TMZ(2,4).**
Fig. 16 zeigt die durch ein zeitlich veränderliches Magnetfeld H modifizierten, elektrisch polarisierbaren Biomaterialien, Polyelektrolytmaterialien, Atomen, Ionen und/oder Moleküle **(epAIMP_{A}')** in der **TMZ(2,2)** und **(epAIMP_{A}')** in der **TMZ(3,2).** Das **epAIMP_{A}** wird translatorisch (a) oder rotatorisch (b) bewegt.
Fig. 17 zeigt die markerfreie, elektrische Detektion von elektrisch polarisierbaren Biomaterialien, Polyelektrolytmaterialien, Atomen, Ionen und/oder Molekülen **(epAIMP)** über dem Trägermaterial **TM** mittels Elektronenstrahlen **(23,24).**

### Beste Ausführungsformen

Trägermaterial umfassend ein Halbleitermaterial, beispielsweise Silizium, mit isolierender Deckschicht **1,** wobei die oberflächennahen elektrostatischen Kräfte durch die lokale Dotierung des Halbleiters mit Donatoren oder Akzeptoren und durch die Dicke der isolierenden Deckschicht bestimmt wird. Es ist in der Regel ausreichend, wenn das das Halbleitermaterial mindestens 1 µm dick und die isolierende Deckschicht ca. 2 bis 3 nm dick ist. Dieses Trägermaterial kann als Biochip verwendet werden.

Durch Anbringen mindestens einer Rückseitenelektrode an dem Halbleitermaterial, kann der Biochip als wiederverwendbarer Filter eingesetzt werden. Dabei ist es durch Änderung der an der Rückseitenelektrode angelegten Spannung möglich, sukzessive verschiedene, am Trägermaterial angelagerte **epAIMP** abzustoßen bzw. abzulösen und nach jedem Ablöseschritt die abgelösten **epAIMPs** zu quantifizieren. Die Quantifizierung kann markerfrei und elektrisch mittels mittels Elektronenstrahlen **(23,24)** aus einer Elektronenquelle **25** durch einem Elektronendetektor **26** erfolgen.

Eine flächendeckende Unterteilung des Trägermaterials mit isolierender Deckschicht in überlappende Bereiche mit unterschiedlichen oberflächennahen elektrostatischen Kräfte, ermöglicht eine spezifische Manipulation, Modifikation und Bewegung verschiedener **epAIMP** auf der Oberfläche des Trägermaterials. Idealerweise ist an jeder Trägermaterialzelle **TMZ(i,j)** ein eigener Rückseitenkontakt angebracht.

### Weitere Ausführungsbeispiele

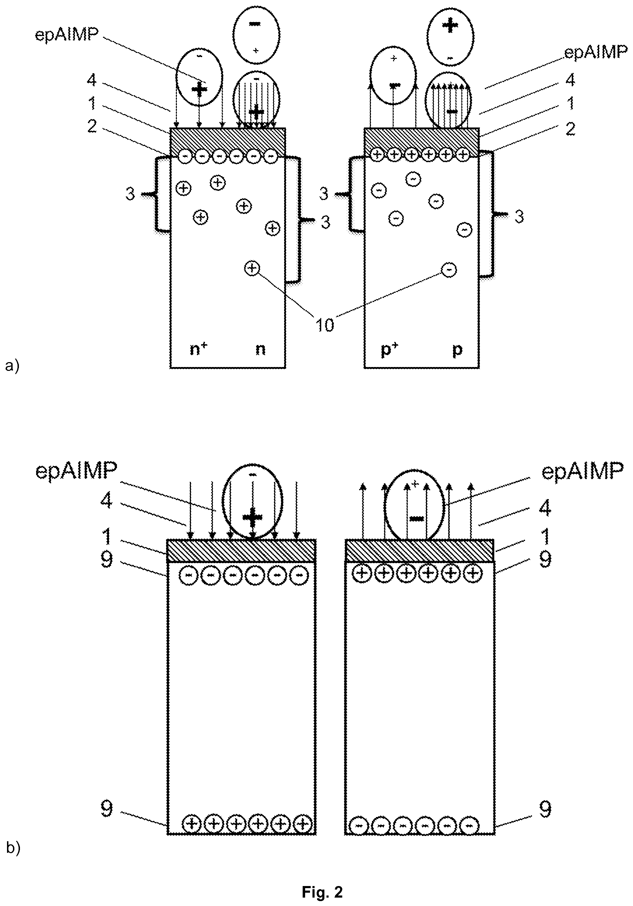

Fig. 2 a) zeigt die Verwendung des erfindungsgemäßen Trägermaterials als halbleitenden, lokal dotierten p-type Halbleiters **p, p⁺** und/oder n-type Halbleiters **n, n⁺** mit vorzugsweise lokal unterschiedlicher Akzeptorkonzentration N_{A} im p-Halbleiter und mit vorzugsweise lokal unterschiedlicher Donatorkonzentration N_{D} im n-type Halbleiter, wobei der Halbleiter auch undotiert sein kann. Auf der Halbleiteroberfläche befindet sich vorzugsweise eine isolierende Deckschicht **1.** Es bilden sich besetzte Grenzflächenzustände der Zahl **G** zwischen der isolierenden Deckschicht **1** und dem n-type Halbleiter bzw. dem p-type Halbleiter aus. Des Weiteren sind im oberflächennahen Bereich des p-type Halbleiters **G** Akzeptoren unabgeschirmt **10** und ionisiert (-) bzw. im n-type Halbleiter **G** Donatoren unabgeschirmt **10** und ionisiert (+). Die besetzten Grenzflächenzustände und die unabgeschirmten Akzeptoren bzw. Donatoren bilden einen asymmetrischen elektrostatischen Dipol **3.** Der Richtungssinn der oberflächennahen, elektrostatischen Kräfte **4** vom asymmetrischen elektrostatischen Dipol **3** über einem p-type Halbleiter **p** und über einem n-type Halbleiter **n** ist entgegengesetzt. Die Stärke der oberflächennahen, elektrostatischen Kraft **4** über dem p-type Halbleiter steigt mit abnehmender Akzeptorkonzentration **N_{A}** und über dem n-Halbleiter steigt mit abnehmender Donatorkonzentration **N_{D}.** Die Eigenschaften der Grenzfläche zwischen dem Halbleitermaterial und der isolierenden Deckschicht bezüglich der Zustandsdichte und Zeitkonstante der Grenzflächenzustände können durch physikalische, chemische oder thermische Vorbehandlung der Halbleiteroberfläche vor dem Aufbringen der isolierenden Deckschicht **1** gezielt eingestellt werden.

Fig. 2b) zeigt die Verwendung des erfindungsgemäßen Trägermaterials als piezoelektrisches Material mit der Polarisationsladung **9** an der Ober- und Unterseite des piezoelektrischen Materials. Auf der Oberfläche des piezoelektrischen Materials befindet sich vorzugsweise eine isolierende Deckschicht **1.** Der Richtungssinn der oberflächennahen, elektrostatischen Kräfte **4** ist durch das Vorzeichen der Polarisationsladung **9** an der Ober- und Unterseite des piezo- oder ferroelektrischen Materials bestimmt. Die Stärke der oberflächennahen, elektrostatischen Kräfte **4** steigt mit der Zahl Polarisationsladungen **9** pro Flächeneinheit bis zu einem materialabhängigen Sättigungswert an. Die Stärke der oberflächennahen, elektrostatischen Kräfte **4** steigt mit zunehmendem Abstand der Polarisationsladungen **9** zwischen der Ober- und Unterseite des piezoelektrischen Materials bis zu einem materialabhängigen Sättigungswert an.

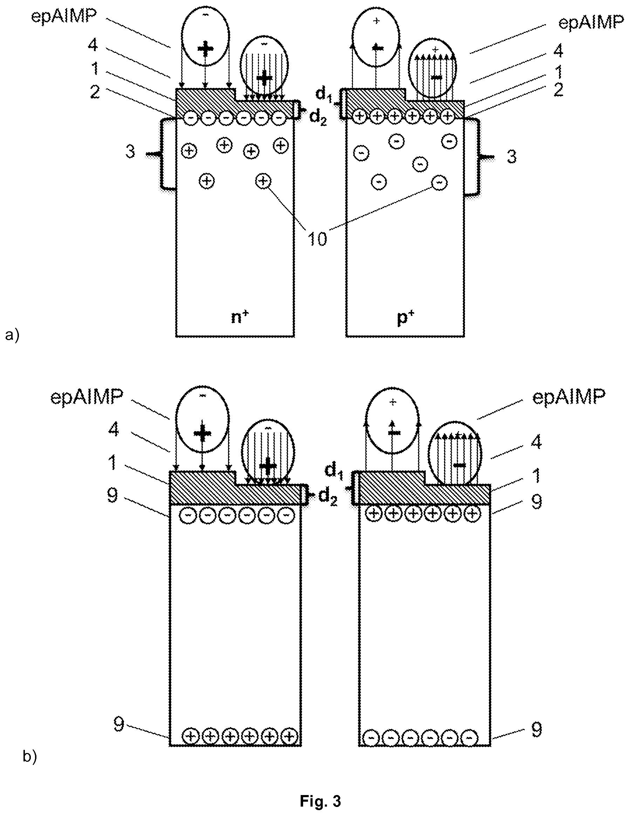

Fig. 3 zeigt ein Trägermaterial **TM** aus halbleitendem (a) und aus piezo- oder ferroelektrischem (b) Material mit strukturierter, isolierender Deckschicht **(1)** mit oberflächennaher elektrostatischer Kraft **(4)** mit gleichem Richtungssinn und unterschiedlicher Stärke.

Neben der Wahl der Spezies **(p** oder **n)** und der Konzentration der Majoritätsladungsträger (N_{A} oder N_{D}) können die oberflächennahen, elektrostatischen Kräfte **(4)** durch lokale Variation der Dicke **dᵢ** der isolierenden Deckschicht **1** lokal variiert werden (Fig. 3a). Die elektrostatischen Kräfte **4** nehmen mit abnehmender Dicke **dᵢ** der isolierenden Deckschicht **1** zu. Die lokale Modifizierung der Dicke **d** der isolierenden Deckschicht **1** kann mittels Photolithographie erfolgen.

Die oberflächennahen, elektrostatischen Kräfte **(4)** oberhalb eines Trägermaterials mit piezoelektrischem Material können durch lokale Variation der Dicke dᵢ der isolierenden Deckschicht **1** lokal variiert werden (Fig. 3b). Die elektrostatischen Kräfte **4** nehmen mit abnehmender Dicke **dᵢ** der isolierenden Deckschicht **1** zu. Die lokale Modifizierung der Dicke **d** der isolierenden Deckschicht **1** kann mittels Photolithographie erfolgen.

Die isolierende Deckschicht **1** kann ein high-k oder low-k Oxid sein. Beispielsweise bei einem Trägermaterial **TM** aus halbleitendem Material kann das entsprechende Oxid des Halbleitermaterials verwendet werden. Bei einem Trägermaterial aus piezoelektrischem Material kann beispielsweise Silizium- oder Aluminiumoxid verwendet werden. Darüber hinaus sind biokompatible Materialien mit einer großen Bandlücke **E_{g},** wie Zinkoxid oder Titandioxid, als isolierende Deckschicht **1** möglich.

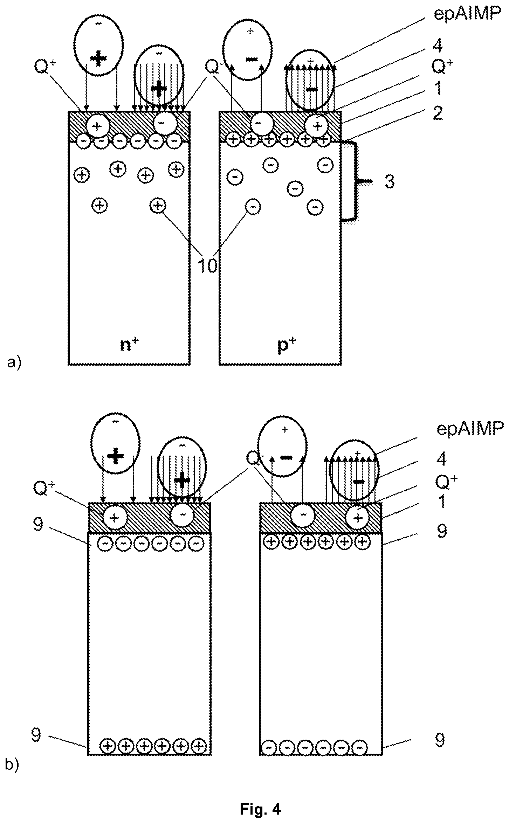

Fig. 4 zeigt die Verwendung von geladenen Störstellen **Q⁺** oder **Q⁻** in der isolierenden Deckschicht **(1)** zur Abschwächung oder Verstärkung der oberflächennahen, elektrostatischen Kräfte und damit zur gezielten Veränderung der auf die **epAIMP** wirkenden Anziehungs- und Abstoßungskräfte.

Die oberflächennahen, elektrostatischen Kräfte **4** von dem asymmetrischen elektrostatischen Oberflächendipol **3** können durch positive (+) Ladungen **Q⁺** und/oder negative (-) Ladungen **Q⁻,** durch sogenannte Oxidladungen in der isolierenden Deckschicht **1** abgeschwächt oder verstärkt werden (Fig. 4 a). So werden z.B. beim Einbringen von negativen Ladungen **Q⁻** in der isolierenden Deckschicht **1** über einen p-type Halbleiter **p** die oberflächennahen, elektrostatischen Kräfte **4** abgeschwächt, wohingegen positive Ladungen **Q⁺** in der isolierenden Deckschicht **1** über einen p-type Halbleiter **p** die oberflächennahen, elektrostatischen Kräfte **4** verstärken. Andererseits werden beim Einbringen von negativen Ladungen **Q⁻** in der isolierenden Deckschicht **1** über einen n-type Halbleiter n die oberflächennahen, elektrostatischen Kräfte **4** verstärkt, wohingegen positive Ladungen **Q⁺** in der isolierenden Deckschicht **1** über einen n-type Halbleiter n die oberflächennahen, elektrostatischen Kräfte **4** abschwächen.

Die oberflächennahen, elektrostatischen Kräfte **4** der Polarisationsladung **9** an der Ober- und Unterseite des piezoelektrischen Materials können durch positive (+) Ladungen **Q⁺** und/oder negative (-) Ladungen **Q⁻,** durch sogenannte geladene Störstellen im Trägermaterial, vorzugsweise in der isolierenden Deckschicht abgeschwächt oder verstärkt werden (Fig. 4 b). So werden z. B. beim Einbringen von negativen Ladungen **Q⁻** in der isolierenden Deckschicht **1** über einem piezoelektrischen Material mit positiver Polarisationsladung **9** an der Oberseite des piezoelektrischen Materials die oberflächennahen, elektrostatischen Kräfte **4** abgeschwächt, wohingegen positive Ladungen **Q⁺** in der isolierenden Deckschicht **1** über einem piezo- oder ferroelektrischen Material mit positiver Polarisationsladung **9** an der Oberseite des piezoelektrischen Materials die oberflächennahen, elektrostatischen Kräfte **4** verstärken. Die verwendeten geladenen Störstellen **Q⁺** und **Q⁻** können optional zusätzlich oder nur im Halbleitermaterial oder im piezoelektrischen Material eingebracht werden.

Die verwendeten geladenen Störstellen **Q⁺** und **Q⁻** können zusätzlich magnetisierbar sein und damit durch ein von außen angelegtes Magnetfeld **14** aktiviert werden.

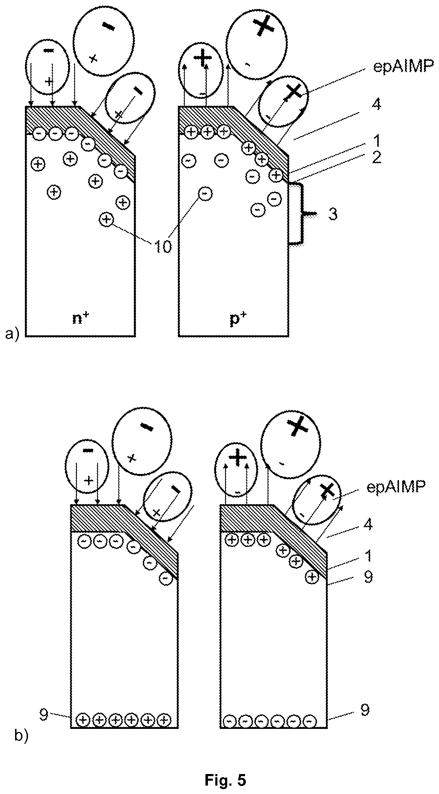

Die Richtung der oberflächennahen, elektrostatischen Kräfte **4** kann durch Strukturierung der Oberfläche des n-type Halbleiters **n** und/oder des p-type Halbleiters **p,** z.B. mittels Photo-, Elektronenstrahl- und/oder lonenstrahllithographie, vorzugweise vor dem Aufbringen der isolierenden Deckschicht **1** modifiziert werden (Fig. 5a). Bei einer unstrukturierten Halbleiteroberfläche ist die oberflächennahe, elektrostatische Kraft **4** vorzugsweise normal zur Oberfläche ausgerichtet. Die Richtung der oberflächennahen, elektrostatischen Kräfte **4** bestimmt die Ausrichtung der **epAIMP** an der Oberfläche des Trägermaterials **TM.**

Die Richtung der oberflächennahen, elektrostatischen Kräfte **4** kann durch Strukturierung der Oberfläche des piezo- oder ferroelektrischen Materials, z.B. mittels Photo-, Elektronenstrahl- und/oder lonenstrahllithographie, vorzugweise vor dem Aufbringen der isolierenden Deckschicht **1** modifiziert werden (Fig. 5b) . Bei einer unstrukturierten Oberfläche des piezoelektrischen Materials ist die oberflächennahe, elektrostatische Kraft **4** vorzugsweise normal zur Oberfläche ausgerichtet. Die Richtung der oberflächennahen, elektrostatischen Kräfte **4** bestimmt die Ausrichtung der **epAIMP** an der Oberfläche des Trägermaterials **TM.**

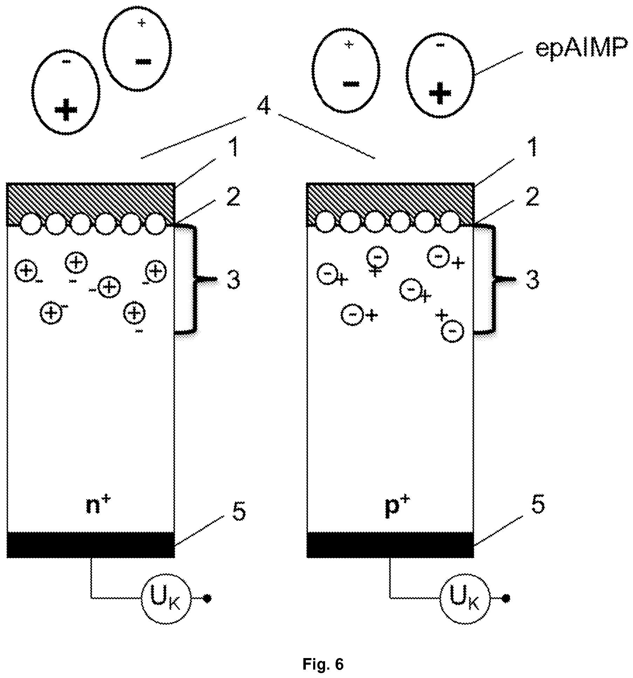

Für die Manipulation der **epAIMP** an der Oberfläche wird an der Rückseite des dotierten Halbleiters eine metallisch leitende Rückseitenelektrode **5** aufgebracht (Fig. 6). An die Rückseitenelektrode **5** wird eine Gleichspannung U_{K} angelegt. Wichtig ist, dass die oberflächennahen, elektrostatischen Kräfte **4** durch das Anlegen einer geeigneten Gleichspannung U_{K} minimiert bzw. nullifiziert werden. In Fig. 6 sind die oberflächennahen, elektrostatischen Kräfte **4** nullifiziert. Beispielsweise im Halbleitermaterial Silizium entspricht die geeignete Gleichspannung **U_{K}** dem Energieabstand zwischen der von der Donatorkonzentration **N_{D}** abhängigen Lage des Ferminiveaus und der Leitungsbandkante **E_{C}** im n-type Halbleiter **n** und/oder dem Energieabstand zwischen der von der Akzeptorkonzentration N_{A} abhängigen Lage des Ferminiveaus und der Valenzbandkante **Ev** im p-type Halbleiter **p.** Vorzugsweise ist die Rückseitenelektrode **5** großflächig aufgebracht. Mit dieser Anordnung können die oberflächennahen, elektrostatischen Kräfte **4** ausgelöscht werden.

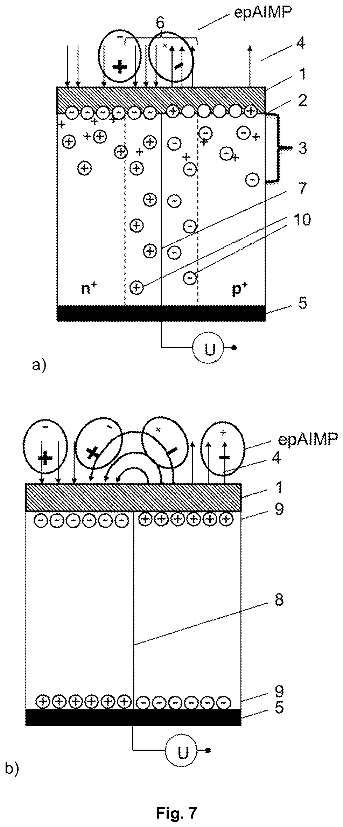

Der Halbleiter wird derart dotiert (Fig. 7a), dass unterschiedlich dotierte Bereiche des Halbleiters an Grenzflächen **7** zusammentreffen. An solchen Grenzflächen **7** bildet sich ein Bereich (Raumladungszone) aus, welcher keine freien Ladungsträger (nur unabgeschirmte Dotieratome **10)** enthält. Senkrecht zur Grenzfläche **7** bildet sich ein elektrisches Feld aus, dessen Maximum in der Grenzfläche **7** liegt und welches am Rand der Raumladungszone Null ist. Für eine zeitabhängige Manipulation (Fig. 7a) der **epAIMP** an der Oberfläche wird an der Rückseite des dotierten Halbleiters eine metallisch leitende Rückseitenelektrode **5** aufgebracht. An die Rückseitenelektrode **5** wird eine Spannung **U** angelegt. Die Spannung **U** kann eine Überlagerung aus einer Wechselspannung und einer Gleichspannung sein. Die oberflächennahen, elektrostatischen Kräfte **4** werden durch das Anlegen der Spannung **U** zeitlich abhängig minimiert und maximiert. Vorzugsweise ist die Rückseitenelektrode **5** großflächig aufgebracht. Die elektrischen Felder senkrecht zur Grenzfläche **7** verlaufen nicht zwingend parallel/antiparallel zum elektrischen Feld, welches durch das Anlegen der Spannung **U** an der Rückseitenelektrode **5** zwischen der Rückseitenelektrode **5** und der Oberfläche des Halbleiters ausgebildet wird. Wichtig ist, dass eine gesonderte zeitabhängige Manipulation der **epAIMP** nahe von Grenzflächen **7** aufgrund der oberflächennahen, elektrostatischen Kräfte im Übergangsbereich **6** möglich ist, da beim Anlegen der Spannung **U** die Verschiebung von freien Ladungsträgern in der Raumladungszone des Halbleiters durch intrinsische elektrische Felder beeinflusst wird.

Das piezo- oder ferroelektrische Material wird derart strukturiert (Fig. 7 b), dass unterschiedliche Bereiche des piezo- oder ferroelektrischen Materials mit unterschiedlicher Polarisationsladung **9** (Domäne) an der Ober- und Unterseite des piezo- oder ferroelektrischen Materials an Grenzfläche **8** zusammentreffen. Die Richtung der oberflächennahen, elektrostatischen Kräfte oberhalb-links und oberhalb-rechts der solcher Grenzflächen **8** ist entgegengesetzt. Die oberflächennahen, elektrostatischen Kräfte oberhalb solcher Grenzflächen **8** verlaufen nicht zwingend normal zur Oberfläche des Trägermaterials, vielmehr gehen sie kontinuierlich ineinander über.

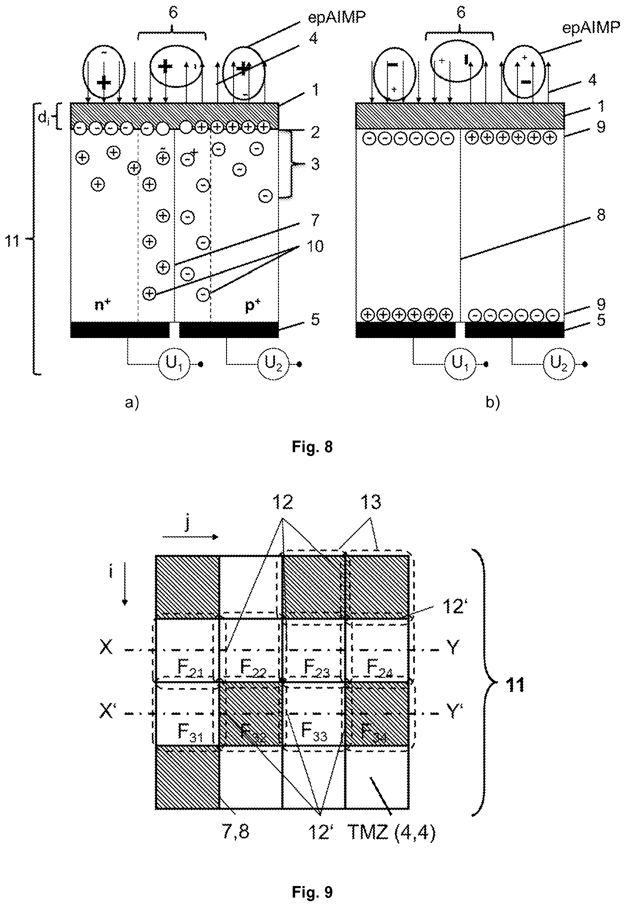

Für eine stark zeitabhängige Manipulation (Fig. 8a) der **epAIMP** an der Oberfläche wird an der Rückseite des dotierten Halbleiters eine strukturierte metallisch leitende Rückseitenelektrode **5** aufgebracht. An die strukturierte Rückseitenelektrode **5** wird eine Spannung **Uᵢ** mit i = 1, ..., m, wobei m die Anzahl der Rückseitenelektroden **5** angibt, angelegt. Die Spannung **Uᵢ** ist eine Überlagerung aus einer Wechselspannung und einer Gleichspannung. Die oberflächennahen, elektrostatischen Kräfte **4** werden durch das Anlegen der Spannungen Uᵢ in den verschiedenen Bereichen unabhängig voneinander kontrolliert. Die Ausbildung von strukturierten, oberflächennahen, elektrostatischen Kräften **4** kann auch durch strukturierte Implantation des Halbleiters, z.B. durch eine um 90° gegeneinander gedrehte Ausrichtung (Crossbar-Array) von streifenförmig implantierten Halbleiterbereichen und von streifenförmig strukturierten Rückseitenelektrodenbereichen **5** eingestellt werden.

Für eine stark zeitabhängige Manipulation (Fig. 8b) der **epAIMP** an der Oberfläche wird an der Rückseite des piezo- oder ferroelektrischen Materials eine strukturierte metallisch leitende Rückseitenelektrode **5** aufgebracht. An die strukturierte Rückseitenelektrode **5** wird eine Spannung **Uᵢ** angelegt. Die Spannung **Uᵢ** ist eine Überlagerung aus einer Wechselspannung und einer Gleichspannung. Die oberflächennahen, elektrostatischen Kräfte **4** werden durch das Anlegen der Spannungen **Uᵢ** in den verschiedenen Bereichen unabhängig voneinander kontrolliert.

Die oberflächennahen, elektrostatischen Kräfte **6** oberhalb der Grenzfläche **8** sind im Übergangsbereich besonders stark und dort können epAIMP besonders stark beeinflusst werden. Das Trägermaterial, bevorzugt die isolierende Deckschicht **1,** kann lokal oder ganzflächig derart gestaltet sein, dass es lokal oder ganzflächig optisch aktiv ist, z.B. durch Verwendung von ZnO und TiO₂ als Material für die isolierende Deckschicht **1.** Bei sehr starker optischer Aktivierung des Trägermaterials und die dadurch erzeugte Wärmeenergie kann das Biomaterial oder das Biomolekül (z. B. Viren oder Bakterien) auch zerstört werden. Wichtig für die optische Aktivierung ist, dass die Photonen- oder Lichtenergie größer als die Bandlücke des Halbleitermaterials, des ferro- oder piezoelektrischen Materials oder des Materials der isolierenden Deckschicht des Trägermaterials ist.

Analog kann die Aktivierung der magnetisierbaren geladenen Störstellen im Trägermaterial durch ein äußeres statisches oder zeitlich veränderliches Magnetfeld erfolgen und die damit erzeugte Wärmeenergie an das Biomaterial bzw. die Biomoleküle weitergegeben werden.

Auf die isolierende Deckschicht **1** auf einem Halbleitermaterial kann bei Verwendung der optischen Aktivierung des Trägermaterials oder die Aktivierung der magnetisierbaren geladenen Störstellen im Trägermaterial oder bei Verwendung unter vakuumähnlichen Bedingungen verzichtet werden. In allen anderen Fällen wird empfohlen, auf dem Halbleitermaterial eine isolierende Deckschicht **1** aufzubringen.

Bei ferro- oder piezoelektrischen Material empfiehlt es sich, eine isolierende Deckschicht **1** aufzubringen, um störende Einflüsse aus der Umgebung zu minimieren.

Fig. 9 zeigt das erfindungsgemäße Trägermaterial **11** in Arraystruktur mit i Zeilen und j Spalten in Draufsicht. Das Trägermaterial **11** wird lückenlos und überlappungsfrei aus einzelnen Trägermaterialzellen **TMZ(i,j),** z.B. quadratische Trägermateriazellen, gebildet. Jede Trägermaterialzelle umfasst ein Halbleitermaterial oder ein piezo- oder ferroelektrisches Material jeweils optional mit isolierender Deckschicht **1** und einer metallisch leitenden Rückseitenelektrode. Die oberflächennahen, elektrostatischen Kräfte **Fᵢⱼ** sind durch Ausbildung eines asymmetrischen elektrostatischen Dipols **3** im Halbleitermaterial oder durch Polarisationsladungen im piezo- oder ferroelektrischen Material, durch die Dicke der isolierenden Deckschicht **1** und durch die an die metallisch leitende Rückseitenelektrode **5** angelegte Spannung **Uij** bestimmt. Da elektrostatische Kräfte langreichweitig sind, ist der Wirkungsbereich **13** der elektrostatischen Kräfte **Fᵢⱼ** größer als die Ausdehnung der einzelnen Trägermaterialzellen **TMZ(i,j).** Der Überlappungsbereich **12** der oberflächennahen elektrostatischen Kräfte zwischen benachbarten Trägermaterialzellen **TMZ(i,j)** des Arrays bei gleicher Richtung der elektrostatischen Kräfte (Fig. 9) bestimmt die translatorische Bewegung der **epAIM.**

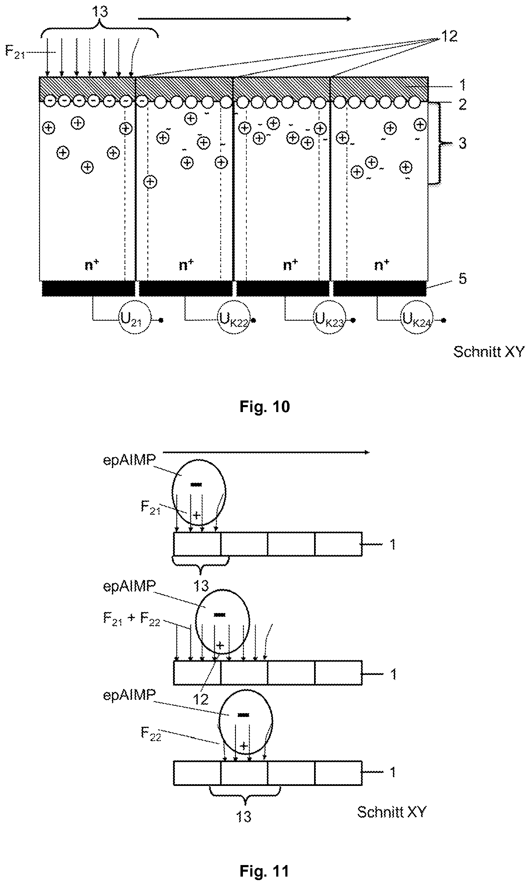

Die Schnittebene **XY** in Fig. 10 kennzeichnet aneinandergrenzende Trägermaterialzellen **TMZ(2,1), TM (2,2), TM (2,3)** und **TM (2,4)** mit gleicher Richtung der oberflächennahen, elektrostatischen Kräften **F₂₁, F₂₂, F₂₃, F₂₄.** Die oberflächennahen, elektrostatischen Kräften **F₂₂, F₂₃** und **F₂₄** sind in der Abbildung durch Anlegen der entsprechenden Kelvin-Spannung **U_{K22}, U_{K23}** und **U_{K24}** nullifiziert. Entlang der Schnittebene **XY** können die überlagerten oberflächennahen, elektrostatischen Kräfte **F₂₁, F₂₂, F₂₃, F₂₄** durch Anlegen äußerer, überlagerter Gleich- und Wechselspannungen **U21, U22, U23** und/oder **U24** an die einzelnen Trägermaterialzellen **TMZ(2,1), TMZ(2,2), TMZ(2,3)** und **TMZ(2,4)** in den einzelnen Überlappungsbereichen **12** zwischen den einzelnen Trägermaterialzellen einen besonders großen Gradienten aufweisen. Das ist darauf zurückzuführen, dass der Gradient der oberflächennahen, elektrostatischen Kräfte **F₂₁, F₂₂, F₂₃, F₂₄** mit gleichen Richtungen außerhalb der Überlappungsbereiche **12** homogen ist und in den Überlappungsbereichen kontinuierlich seinen Wert ändern kann, falls an einer oder mehreren angrenzenden Trägermaterialzellen **TMZ(i,j)** die entsprechende Kelvin-Spannung **U_{Kij}** zur Minimierung/Nullifizierung der oberflächennahen, elektrostatischen Kraft **Fᵢⱼ** angelegt wird.

Damit bestimmen hauptsächlich die Überlappungsbereiche **12** die Dynamik der translatorischen Bewegung entlang der Schnittebene **XY.**

Fig. 11 zeigt die translatorische Bewegung eines **epAIMP M** oberhalb der isolierenden Schicht **1** der Trägermaterialzelle entlang der Schnittebene XY, deren Kräfte beim Anlegen einer äußeren Spannung gleich gerichtet sind. Im oberen Bildteil wirkt die Kraft **F₂₁** auf das **epAIMP M** über der Trägermaterialzelle **TMZ(2,1).** Durch Ändern der an die Trägermaterialzelle **TMZ(2,2)** angelegten Spannung **U₂₂** wird die Gradient der elektrostatischen Kraft **F₂₁+F₂₂** im Überlappungsbereich **12** zwischen **TMZ(2,1)** und **TMZ(2,2)** im Vergleich zur Kraft **F₂₁** der **TMZ(2,1)** verstärkt, so dass sich das **epAIMP** translatorisch zum Überlappungsbereich **12** bewegt (mittlerer Bildteil). Zum Abschluß der gezeigten translatorischen Bewegung von der **TMZ(2,1)** zur **TMZ(2,2),** wird an die **TMZ(2,1)** die Kelvin-Spannung **U_{K12}** angelegt, so dass nur noch über der **TMZ(2,2)** eine elektrostatische Kraft **F₂₂** wirkt.

Der Überlappungsbereich **12'** der oberflächennahen elektrostatischen Kräfte zwischen benachbarten Trägermaterialzellen **TMZ(i,j)** des Arrays bei unterschiedlicher Richtung der elektrostatischen Kräfte in Fig. 9 bestimmt die rotatorische Bewegung der **epAIMP.**

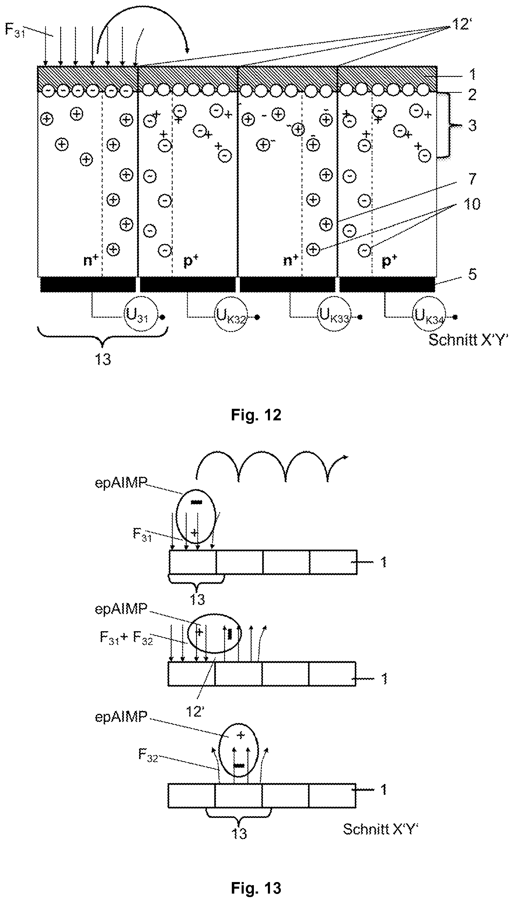

Die Schnittebene **X'Y'** in Fig. 12 kennzeichnet aneinandergrenzende Trägermaterialzellen **TMZ(3,1), TM (3,2), TM (3,3)** und TM **(3,4)** mit verschiedener Richtung der oberflächennahen, elektrostatischen Kräften **F₃₁, F₃₂, F₃₃, F₃₄.** Die oberflächennahen, elektrostatischen Kräften **F₃₂, F₃₃** und **F₃₄** sind in der Abbildung durch Anlegen der entsprechenden Kelvin-Spannung **U_{K32}, U_{K33}** und **U_{K34}** minimiert. Diese Trägermaterialzellen sind in Fig. 12 in der Seitenansicht gezeigt. Entlang der Schnittebene **X'Y'** kann durch Anlegen einer äußeren Spannung **U₃₁, U_{K32}, U_{K33}** und **U_{K34}** an die einzelnen Trägermaterialzellen **TMZ(3,1), TM (3,2), TM (3,3)** und **TM(3,4)** der Gradient der überlagerten oberflächennahen elektrostatischen Kräfte **F₃₁, F₃₂, F₃₃, F₃₄ in** den Überlappungsbereichen **12'** der oberflächennahen, elektrostatischen Kräfte benachbarter Trägermaterialzellen besonders groß sein. Damit bestimmen hauptsächlich die Überlappungsbereiche **12'** die Dynamik der rotatorischen Bewegung entlang der Schnittebene **X'Y'.**

Fig. 13 zeigt schematisch die Rotation eines **epAIMP** aufgrund der oberflächennahen, elektrostatischen Kräfte.

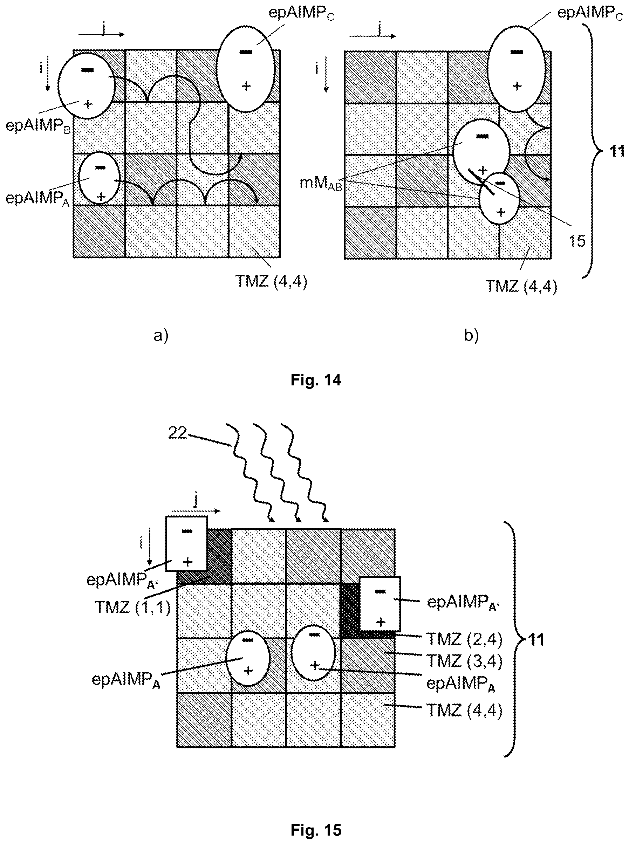

Fig. 14 zeigt das erfindungsgemäße Trägermaterial **11** in Arraystruktur mit i Zeilen und j Spalten in Draufsicht. In Fig. 14a) sind an den Trägermaterialzellen **TMZ(3,1), TMZ(1,1)** und **TMZ(1,4)** die **epAIMP M_{A}, M_{B}** und **M_{C}** lokalisiert. Das **epAIMP** M_{A} wird von der **TMZ(3,1)** nach **TMZ(3,4)** rotatorisch und das **epAIMP M_{B}** wird von **TMZ(1,1)** nach **TMZ(2,3)** rotatorisch, von **TMZ(2,3)** nach **TMZ(3,3)** translatorisch und von **TMZ(3,3)** nach **TMZ(3,4)** rotatorisch bewegt. Die beiden **epAIMP M_{A}** und **M_{B}** bilden eine Verbindung **15** (Fig. 14b)). Danach wird das **epAIMP M_{C}** von **TMZ(1,4)** nach **TMZ(3,4)** rotatorisch bewegt. Die verbundenen **epAIMP M_{A}, M_{B}** und **M_{C}** können eine molekulare Maschine **mM** bilden.

Fig. 15 zeigt das an der **TMZ(1,1)** lokalisierte, temperaturempfindliche **epAIMP_{A}** nach der Modifikation zu **epAIMP_{A}'** und das an der **TMZ(2,4)** lokalisierte temperaturempfindliche **epAIMP_{A}** nach der Modifikation zu **epAIMP_{A}'.** Die Trägermaterialzellen **TMZ(i,j)** des Trägermaterials **11** umfassen halbleitende und/oder piezoelektrische Materialien mit unterschiedlichen Bandlücken **E_{g}(i,j).** Dadurch kann bei großflächiger Beleuchtung des Trägermaterials **11** mit elektromagnetischen Wellen **22,** vorzugsweise mit Licht, von der Rückseite und/oder von der Vorderseite des Trägermaterials **11** mit einer Energie kleiner als die Bandlücke der Trägermaterialzellen **TMZ(3,2)** und **TMZ(3,3)** und mit einer Energie größer als die Bandlücke der Trägermaterialzellen **TMZ(1,1)** und **TMZ(2,4)** Licht in der **TMZ(1,1)** und **TMZ(2,4)** lokal absorbiert und in Wärmeenergie umgewandelt werden.

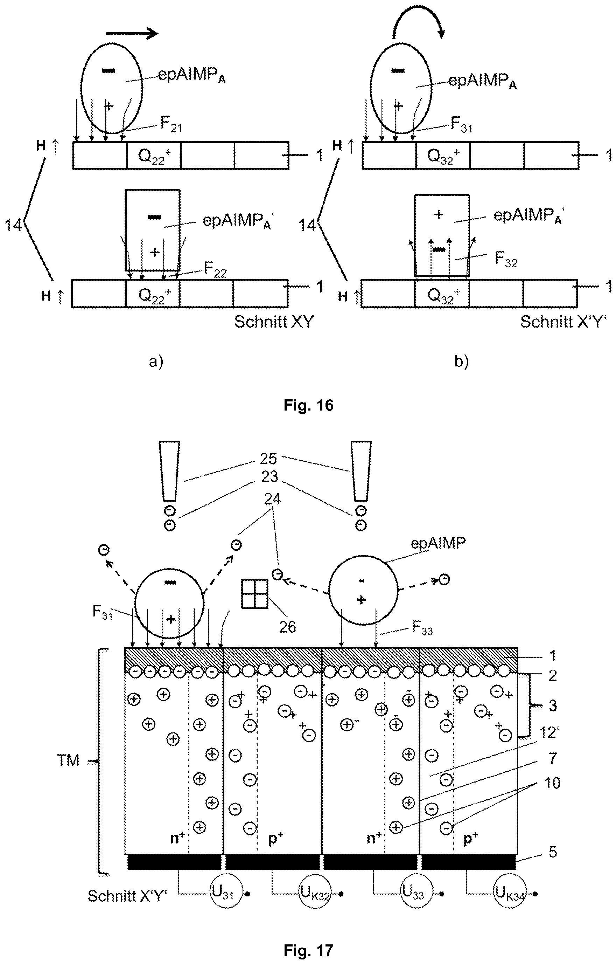

Fig. 16a) zeigt die Modifikation der an der **TMZ(2,1)** lokalisierten **epAIMP_{A}** nach einer translatorischer Bewegung von der **TMZ(2,1)** zur **TMZ(2,2)** entlang dem Schnitt **XY** aus Fig. 11.

Fig. 16b) zeigt die Modifikation der an der **TMZ(3,1)** lokalisierten **epAIMP_{A}** nach einer rotatorischer Bewegung von **TMZ(3,1)** zur **TMZ(3,2)** entlang dem Schnitt **X'Y'** aus Fig. 13. Die in die isolierende Deckschicht **1** der **TMZ(2,2)** und der **TMZ(3,2)** eingebrachten geladenen Störstellen **Q⁺** beeinflussen in dieser Anordnung vorzugsweise die oberflächennahen elektrostatischen Kräfte **F₂₂** (Fig. 16a)) und F₃₂ (Fig. 16b)) nicht und sind durch ein von außen angelegtes Magnetfeld **H** magnetisierbar. Wird von außen ein zeitlich veränderliches Magnetfeld **H** angelegt, dann ändert sich zeitlich veränderlich die Magnetisierungsrichtung der Störstellen **Q⁺** und es wird lokal Wärmeenergie beim Ummagnetisieren entwickelt, die zur Modifikation von temperaturempfindlichen **epAIMP_{A}** an den **TMZ(2,2)** und **TMZ(3,2)** genutzt wird.

Falls die elektrisch polarisierbaren Biomaterialien, Polyelektrolytmaterialien, Atome, Ionen oder Moleküle zusätzlich auch magnetisierbar sind, dann kann die Manipulation, Modifikation und Bewegung zusätzlich durch oberflächennahe magnetostatische Kräfte erfolgen, so wie sie beispielsweise in der Nähe von magnetisierbaren Störstellen **Q_{M}** auftreten.

Fig. 17 zeigt die markerfreie, elektrische Detektion von elektrisch polarisierbaren Biomaterialien, Polyelektrolytmaterialien, Atomen, Ionen und/oder Molekülen **(epAIMP)** in Pulvern, Flüssigkeiten, Gasen mittels Primärelektronenstrahlen **(23)** aus mindestens einer Elektronenquelle **(25),** wobei die Streuung der Sekundärelektronen **(24),** die durch Auftreffen der Primärelektronen **(23)** auf **epAIMPs** gebildet werden, von der elektrischen Polarisation der **epAIMP** abhängt und mittels eines Elektronendetektors **(26)** bestimmt wird, wobei die Streuung der Sekundärelektronen durch die elektrische Polarisation der **epAIMP** mittels Verändern der oberflächennahen elektrostatischen Kräfte am Trägermaterial **TM** unter Ausnutzung der Manipulation, Modifikation und translatorischen und rotatorischen Bewegung der **epAIMP** eingestellt wird.

### Bezugszeichen

- TM: Trägermaterial
- PEE: Einkomponentige Polyelektrolytsysteme
- PEM: Polyelektrolytmultischicht
- PEC: Polyelektrolytkomplexartikel
- PEL: Polyelektrolytmaterial
- PEL-1: kontrolliert inerte Passivierungsschicht für Biomaterialien, -fluide oder Zellen
- PEL-2: aktiv bindende Schicht für Biomaterialien, -fluide oder Zellen
- PEL-3: biozide Schicht für Biomaterialien, Biofluide oder Zellen
- BM: Biomaterial
- epAIM: Elektrisch polarisierbare Atome, Ionen und /oder Moleküle
- epAIMP: BM, epAIM, PEE, PEC, PEL, PEM, M, M_{A}, M_{B}, Mc, M', M_{A}', M_{B}', M_{C}', mM
- M, M_{A}, M_{B}, M_{C}: elektrisch polarisierbare atomare, ionische und/oder molekulare Komponenten
- M', M_{A}', M_{B}',: modifizierte Komponenten M, M_{A}, M_{B}, M_{C}
- M_{C}' mM: Molekulare Maschine
- TMZ(i,j): Trägermaterialzelle **TMZ(i,j)** in i-ter Zeile und j-ter Spalte des in Array angeordneten Trägermaterials **11**
- 1: Isolierende Deckschicht
- 2: Grenzflächenladung
- 3: Asymmetrischer elektrostatischer Oberflächendipol
- 4: Oberflächennahe, elektrostatische Kraft
- 5: Metallisch leitende Rückseitenelektrode
- 6: Oberflächennahe, elektrostatische Kraft im Übergangsbereich
- 7: Grenze zwischen p-type Halbleiter **p** und n-type Halbleiter **n**
- 8a: Grenze zwischen Domänen mit verschiedener Polarisationsladung
- 8b: Grenze zwischen Domänen mit entgegengesetzt gerichteten intrinsischen elektrischen Feld
- 9: Polarisationsladung
- 10: Unabgeschirmte Dotanden im Halbleiter
- 11: Trägermaterial ausgeführt als Array mit flächiger Anordnung von Trägermaterialzellen **TMZ(i, j)**
- 12: Überlappungsbereich der oberflächennahen, elektrostatischen Kräfte zwischen benachbarten Trägermaterialzellen (i, j) des Arrays 11, bei gleicher Richtung der elektrostatischen Kräfte ohne angelegte Spannung
- 12': Überlappungsbereich der oberflächennahen, elektrostatischen Kräfte zwischen benachbarten Trägermaterialzellen TMZ(i, j) des Arrays 11, bei unterschiedlicher Richtung der elektrostatischen Kräfte ohne angelegte Spannung
- 13: Wirkungsbereich der oberflächennahen elektrostatischen Kraft Fᵢⱼ
- 14: von außen angelegtes Magnetfeld **H**
- 15: Verbindung zwischen zwei elektrisch polarisierbaren Atomen, Ionen und Molekülen
- 22: elektromagnetische Wellen, vorzugsweise monochromatisches Licht
- 23: Primärelektronenstrahl
- 24: Sekundärelektronenstrahl
- 25: Elektronenquelle
- 26: Elektronendetektor
- p, p⁺: Loch-Konzentration p⁺>p in Halbleitern mit Löchern als Majoritätsladungsträger
- n, n⁺: Elektronen-Konzentration n⁺>n in Halbleitern mit Elektronen als Majoritätsladungsträger
- N_{A}: Akzeptorkonzentration im p-type Halbleiter
- N_{D}: Donatorkonzentration im n-type Halbleiter
- dᵢ: Dicke der isolierenden Deckschicht i, i=1,2,...
- G: Zahl der besetzten Grenzflächenzustände zwischen isolierender Deckschicht und p-type oder n-type Halbleiter
- Dᵢⱼ: Grenzflächenzustandsdichte in der Trägermaterialzelle **TMZ(i,j)**
- τᵢⱼ: Zeitkonstante der Grenzflächenzustände in der Trägermaterialzelle **TMZ(i,j)**
- E_{F}: Fermienergie
- E_{C}: Leitungsbandkante
- E_{V}: Valenzbandkante
- E_{g}, E_{g}(i,j): Bandlücke des Halbleitermaterials oder des piezo- oder ferroelektrischen Materials der Trägermaterialzelle **TMZ(i,j)** oder der isolierenden Deckschicht **1**
- XY, X'Y': Schnittebene durch das Array **11** zur Verdeutlichung der Translation bzw. Rotation
- Fᵢⱼ: oberflächennahe elektrostatische Kraft F der Trägermaterialzelle **TMZ(i, j)**
- U_{K}, U_{K1}, U_{K2},: Gleichspannung (Kelvin-Spannung)
- U_{Ki}, U, U₁, U₂, Uᵢ,: überlagerte Gleichspannung und Wechselspannung an der
- Uᵢⱼ: metallisch leitenden Rückseitenelektrode des Trägermaterials **TM** bzw. an der metallisch, leitenden Rückseitenelektrode der Trägermaterialzelle **TMZ(i, j)**
- Q⁺, Q⁻, Qᵢⱼ⁺, Qᵢⱼ⁻: geladene Störstellen in der isolierenden Schicht **1** im Trägermaterial
- , Q_{M}: bzw. in der Trägermaterialzelle **TMZ(i,** j), auch magnetisierbar (Q_{M})

## Patentansprüche

1. Trägermaterial zur Modifikation, Manipulation und Bewegung von elektrisch polarisierbaren Biomaterialien, Polyelektrolytmaterialien, Atomen, Ionen und/oder Molekülen (epAIMP), umfassend halbleitende oder piezoelektrische Materialien, sowie eine isolierende Oberflächendeckschicht auf der Oberfläche der halbleitenden oder piezoelektrischen Materialien, **dadurch gekennzeichnet, dass** durch die Oberflächendeckschicht hindurch elektrostatische Kräfte auf die epAIMP einwirken, wobei die elektrostatischen Kräfte mit einer Reichweite bis zu 100 nm erzeugt und in ihrer Stärke bestimmt werden:
a. durch den, aufgrund der Besetzung der Grenzflächenzustände zwischen dem halbleitenden Material und der Oberflächendeckschicht, wobei die Zeitkonstante der Grenzflächenzustände (Tᵢⱼ) im Bereich von wenigen Nanosekunden bis Sekunden liegt oder die Grenzflächenzustandsdichte (Dᵢⱼ) von 10⁷ bis 10¹⁴ cm⁻² eV⁻¹ variiert, sowie der oberflächennahen Dotierung des halbleitenden Materials mit Volumenladungsdichten von 10¹⁵ bis 10²¹ e/cm³ und Flächenladungsdichten von 10¹⁰ bis 10¹⁴ e/cm², entstehenden asymmetrischen elektrostatischen Dipol, wobei die Flächenladungsdichte im piezoelektrischen Material 10¹³ bis 10¹⁵ e/cm² beträgt, oder
b. durch die Zahl der oberflächennahen Polarisationsladungen pro Flächeneinheit und die Dicke des piezoelektrischen Materials
und wobei die elektrostatischen Kräfte in ihrer Stärke weiterhin durch Art und Dicke des Materials der Oberflächendeckschicht bestimmt werden und die oberflächennahen elektrostatischen Kräfte des Trägermaterials von der Umgebung unabhängig sind und die oberflächennahen elektrostatischen Kräfte die anderen Oberflächeneigenschaften des Trägermaterials nicht verändern und die Dicke der isolierenden Deckschicht (1) kleiner als 20 nm, bevorzugt kleiner als 5 nm ist und besonders bevorzugt zwischen 2 und 3 nm liegt.

2. Trägermaterial nach Anspruch 1, **dadurch gekennzeichnet, dass** mindestens eine metallisch leitende Rückseitenelektrode (5) auf der der Oberflächendeckschicht gegenüberliegenden Seite des Trägermaterials angeordnet ist.

3. Trägermaterial nach Anspruch 2, **dadurch gekennzeichnet, dass** die Rückseitenelektrode strukturiert ist.

4. Trägermaterial nach Anspruch 1, **dadurch gekennzeichnet, dass** im Trägermaterial, in der isolierenden Deckschicht (1) oder im oberflächennahen Bereich der halbleitenden oder piezoelektrischen Materialien, magnetisierbare Störstellen (QM) eingebracht sind.

5. Trägermaterial nach Anspruch 1, **dadurch gekennzeichnet, dass** das Trägermaterial
a. ein Halbleitermaterial umfasst und dass einzelne geladene Störstellen (Q+, Q-, Q+ij, Q-ij) lokal in den Halbleiter eingebracht sind,
oder
b. ein piezoelektrisches Material umfasst und dass geladene Störstellen (Q+, Q-, Q+ij, Q-ij) in das piezoelektrische Material eingebracht sind,
um die oberflächennahen, elektrostatischen Kräfte (Fij) zu beeinflussen.

6. Trägermaterial nach Anspruch 2, **dadurch gekennzeichnet, dass** sich mindestens ein Übergangsbereich zwischen zwei benachbarten Gebieten mit jeweils umgekehrter Richtung der oberflächennahen, elektrostatischen Kräfte im Trägermaterial ausbildet, um durch Anlegen einer Spannung an eine oder mehrere Rückseitenelektroden des Trägermaterials die Manipulation, Modifikation und Bewegung von elektrisch polarisierbaren Biomaterialien, Polyelektrolytmaterialien, Atomen, Ionen und/oder Molekülen (epAIMP) zeitlich und räumlich veränderlich zu gestalten.

7. Trägermaterial nach Anspruch 1, angeordnet als Arraystruktur (11), **dadurch gekennzeichnet, dass** das Trägermaterial einzelne Trägermaterialzellen (i,j) aufweist, die flächig durch verschiedene Polygone gebildet werden, wobei die Eckpunkte der Polygone, geradlinig und/oder gekrümmt paarweise miteinander verbunden sind.

8. Trägermaterial nach Anspruch 7, **dadurch gekennzeichnet, dass** jede oder einzelne Trägermaterialzellen (i,j) mit einer eigenen metallisch leitenden Rückseitenelektrode 5 ausgestattet sind.

9. Trägermaterial nach Anspruch 7, **dadurch gekennzeichnet, dass** in einzelnen Trägermaterialzellen, in der isolierenden Deckschicht (1) oder im oberflächennahen Bereich der halbleitenden oder piezoelektrischen Materialien, magnetisierbare Störstellen (QM) eingebracht sind.

10. Verwendung des Trägermaterials nach einem der Ansprüche 2, 3 oder 6 zur Manipulation von elektrisch polarisierbaren Biomaterialien, Polyelektrolytmaterialien, Atomen, Ionen und/oder Molekülen (epAIMP) auf der Oberfläche des Trägermaterials (TM) oder zwischen benachbarten Trägermaterialzellen (TMZ) nach einem der Ansprüche 7 bis 9, **dadurch gekennzeichnet,**
a. **dass** eine Spannung (U, Ui, Uij, UK) an mindestens eine der Rückseitenelektroden (5) des Trägermaterials (TM) oder der
Trägermaterialzellen (TMZ) angelegt oder verändert wird,
oder
b. **dass** das Trägermaterial mit einer konstanten oder zeitlich veränderlichen elektromagnetischen Welle (22) mit einer Photoenergie, die größer als die Bandlücke des Halbleitermaterials, des piezoelektrischen Materials oder des Materials der isolierenden Deckschicht ist, bestrahlt wird,
so dass freie Ladungsträger im Halbleitermaterial oder Polarisationsladungen im piezoelektrischen Material umverteilt werden und die Stärke und/oder Richtung der oberflächennahen, elektrostatischen Kräfte F(i,j) der Trägermaterialzelle TMZ(i,j) verändert wird.

11. Verwendung des Trägermaterials nach einem der Ansprüche 2, 3 oder 6 oder Trägermaterialzellen nach einem der Ansprüche 7 bis 9 zur Modifikation von elektrisch polarisierbaren Biomaterialien, Polyelektrolytmaterialien, Atomen, Ionen und/oder Molekülen (epAIMP), **dadurch gekennzeichnet, dass**
a. einzelne Trägermaterialzellen (i,j) oder das Trägermaterial (TM) mit elektromagnetischen Wellen (22), vorzugsweise mit Licht, dessen Energie
größer als die Bandlücke des Trägermaterials bzw.der oder einzelner Trägermaterialzellen ist, bestrahlt werden
oder
b. ein äußeres magnetisches Feld (14) zur Ummagnetisierung der magnetisierbaren Störstellen (QM), vorzugsweise ein äußeres magnetisches Wechselfeld, angelegt wird,
oder
c. an die Rückseitenelektrode (5) eine elektrische Gleichspannung und/oder Wechselspannung (Ui, Uij) zur Be-und Entladung der geladenen Ladungstraps/Störstellen (Q+, Q-, Q+ij, Q-ij) angelegt wird,
so dass die Stärke der Änderung der chemischen und/oder physikalischen Eigenschaften der epAIMP an den einzelnen Trägermaterialzellen (i,j) oder dem Trägermaterial von der Intensität der elektromagnetischen Wellen (22), der Stärke des äußeren magnetischen Feld (14) oder der Stärke der elektrischen Gleichspannung und Wechselspannung (Ui, Uij) abhängt und dadurch gesteuert werden kann.

12. Verwendung des Trägermaterials (11) nach einem der Ansprüche 2, 3 oder 6 zur translatorischen oder rotatorischen Bewegung von elektrisch polarisierbaren Biomaterialien, Polyelektrolytmaterialien, Atomen, Ionen und/oder Molekülen (epAIMP) auf oder an der Oberfläche des Trägermaterials (TM) oder zwischen benachbarten Trägermaterialzellen (i,j)nach den Ansprüchen 7 bis 9, in denen die Richtung der oberflächennahen, elektrostatischen Kräfte (Fij) vorzugsweise gleich gerichtet oder vorzugsweise entgegengesetzt gerichtet ist, **dadurch gekennzeichnet, dass** die Stärke und Richtung der oberflächennahen, elektrostatischen Kräfte (Fij)
a. durch Anlegen oder Verändern der angelegten Gleichspannung und/oder Wechselspannung (Ui, Uij) an die Rückseitenelektrode (5) einzelner Trägermaterialzellen,
oder
b. durch Bestrahlung mit elektromagnetischen Wellen (22), vorzugsweise mit Licht, dessen Energie größer als die Bandlücke (Eg) des Trägermaterials (TM) bzw. der oder einzelner Trägermaterialzellen (i,j) ist,
verändert wird.

13. Verwendung des Trägermaterials (11) nach einem der Ansprüche 6 bis 9 zur Herstellung von molekularen Maschinen (mM), **dadurch gekennzeichnet, dass** sich elektrisch polarisierbare Biomaterialien, Atome, Ionen oder Moleküle an einzelnen Trägermaterialzellen TMZ(i,j) des Trägermaterials (11)befinden, und einzeln, d.h. zeitlich separat hintereinander, oder komplett oder teilweise gleichzeitig auf oder in der Nähe der Oberfläche des Trägermaterials mit Arraystruktur von einer Trägermaterialzelle **TMZ(i,j)** zu einer angrenzenden Trägermaterialzelle TMZ(i-1,j), TMZ(i+1,j), TMZ(i,j-1) oder TMZ(i, j+1) bewegt werden können, wobei die Art der Bewegung durch die oberflächennahen, elektrostatischen Kräfte Fij der Trägermaterialzellen TMZ(i,j) bestimmt wird, indem eine translatorische Bewegung der epAIMP durch einen Überlappungsbereich (12) der oberflächennahen elektrostatischen Kräfte zwischen benachbarten Trägermaterialzellen TMZ (i,j) des Arrays bei gleicher Richtung der elektrostatischen Kräfte erfolgt und eine rotatorische Bewegung der epAIMP durch einen Überlappungsbereich (12') der oberflächennahen elektrostatischen Kräfte zwischen benachbarten Trägermaterialzellen TMZ (i,j) des Arrays bei unterschiedlicher Richtung der elektrostatischen Kräfte erfolgt.

14. Verwendung des Trägermaterials (11) nach einem der Ansprüche 1 bis 9 in einem in-situ-Sensor für elektrisch polarisierbaren Biomaterialien, Polyelektrolytmaterialien, Atomen, Ionen und/oder Molekülen (epAIMP) in Flüssigkeiten und Gasen, wobei die Sensoroberfläche, an der die Flüssigkeiten oder Gase mit den zu detektierenden epAIMP vorbeiströmen, durch das Trägermaterial oder das Trägermaterial mit Arraystruktur gebildet wird.

15. Verwendung des Trägermaterials (11) nach einem der Ansprüche 1 bis 9 als Filter für elektrisch polarisierbare Atome, Ionen und/oder Moleküle in Gasen, Flüssigkeiten und Pulvern, wobei die Trägermaterialzellen TMZ(i,j) in Siebstruktur ausgeführt sind und durch die veränderlichen elektrostatischen Kräfte F(i,j) unter Ausnutzung der Ansprüche 10 bis 12 die Filtereigenschaften einzelner Trägermaterialzellen TMZ(i,j) kontrolliert werden können.

## Claims

1. Carrier material for modification, manipulation and movement of electrically polarizable biomaterials, polyelectrolyte materials, atoms, ions and/or molecules (epAIMP), comprising semiconducting or piezoelectric materials, and an insulating surface covering layer on the surface of the semiconducting or piezoelectric materials**characterized in that** through the surface covering layer electrostatic forces act on the epAIMP, wherein the electrostatic forces are generated with a range of up to 100 nm and are determined in their strength:
a. by the, due to the occupation of the interface states between the semiconducting material and the surface covering layer, where the time constant of the interfacial states (Tij) is in the range of a few nanoseconds to seconds, or the interfacial state density (Dij) varies from 10⁷ to 10¹⁴ cm⁻² eV⁻¹ as well as the near-surface doping of the semiconducting material with volume charge densities of 10¹⁵ to 10²¹ e/cm3 and surface charge densities of 10¹⁰ to 10¹⁴ e/cm2, resulting in an asymmetric electrostatic dipole, the surface charge density in the piezoelectric material being 10¹³ to 10¹⁵ e/cm², or
b. by the number of near-surface polarization charges per unit area and the thickness of the piezoelectric material and wherein the electrostatic forces are further determined in their strength by the type and thickness of the material of the surface covering layer and the near-surface electrostatic forces of the substrate material are independent of the environment and the near-surface electrostatic forces do not change the other surface properties of the substrate material and the thickness of the insulating cover layer (1) is less than 20 nm, preferably less than 5 nm, and particularly preferably between 2 and 3 nm.

2. Carrier material according to claim 1, **characterized in that** at least one metallic conductive backside electrode (5) is arranged on the side of the substrate opposite to the surface covering layer.

3. Carrier material according to claim 2, **characterized in that** the back electrode is structured.

4. Carrier material according to claim 1, **characterized in that** in the carrier material, in the insulating cover layer (1) or in the region near the surface of the semiconducting or piezoelectric materials, magnetizable imperfections (QM) are introduced.

5. A carrier material according to claim 1, **characterized in that** the carrier material comprises
a. semiconductor material and that individual charged impurities (Q+, Q-, Q+ij, Q-ij) are locally introduced into the semiconductor, or
b. a piezoelectric material and that charged impurities (Q⁺, Q-, Q+ij, Q-ij) are introduced into the piezoelectric material
in order to influence the near-surface electrostatic forces (Fij)

6. Carrier material according to claim 2,**characterized in that** at least one transition region is formed between two adjacent areas, each with reversed direction of the near-surface electrostatic forces is formed in the carrier material, in order to make the manipulation, modification and movement of electrically polarizable biomaterials, polyelectrolyte materials, atoms, ions and/or molecules (epAIMP) variable in time and space by applying a voltage to one or more back electrodes of the carrier material.

7. A carrier material according to claim 1, arranged as an array structure (11), **characterized in characterized in that** the carrier material has individual carrier material cells (i,j) which are formed areally by different polygons, the corner points of the polygons are connected to one another in pairs in a rectilinear and/or curved manner.

8. Carrier material according to claim 7, **characterized in that** each or individual carrier material cells (i,j) are provided with their own metallically conductive back electrode 5.

9. Carrier material according to claim 7, **characterized in that** magnetizable imperfections (QM) are introduced in individual carrier material cells, in the insulating cover layer (1) or in the surface-near region of the semiconducting or piezoelectric materials.

10. Use of the carrier material according to any one of claims2, 3 or 6for manipulation of electrically polarizable biomaterials, polyelectrolyte materials, atoms, ions and/or molecules (epAIMP) on the surface of the carrier material (TM) or between adjacent carrier material cells (TMZ)according to any one of claims 7 to 9, **characterized in,**
a. **that** a voltage (U, Ui, Uij, UK) is applied to at least one of the back electrodes (5) of the carrier material (TM) or the carrier material cells (TMZ) is applied or changed, or
b. **that** the carrier material is irradiated with a constant or time-varying electromagnetic wave (22) with a photoenergy which is larger than the bandgap of the semiconductor material, the piezoelectric material or the material of the insulating covering layer
so that free charge carriers in the semiconductor material or polarization charges in the piezoelectric material are redistributed and the strength and/or direction of the near-surface electrostatic forces F(i,j) of the carrier material cell TMZ(i,j) are changed.

11. Use of the carrier material according to one of claims 2, 3 or 6 or Carrier material cells according to any one of claims 7 to 9 for the modification of electrically polarizable biomaterials, polyelectrolyte materials, atoms, ions and/or molecules (epAIMP), **characterized in that**
a. individual carrier material cells (i,j) or the carrier material (TM) are treated with electromagnetic waves (22), preferably with light whose energy is is greater than the band gap of the carrier material or of the or individual carrier material cells, respectively, are irradiated
or
b. an external magnetic field (14) for remagnetizing the magnetizable interference points (QM), preferably an external alternating magnetic field, is applied,
or
c. an electrical DC voltage and/or AC voltage (Ui, Uij) is applied to the back electrode (5) for charging and discharging the charged charge traps/interference sites (Q+, Q-, Q+ij, Q-ij) is applied,
such that the magnitude of the change in the chemical and/or physical properties of the epAIMP at the individual carrier material cells (i,j) or the carrier material depends on the intensity of the electromagnetic waves (22), the strength of the external magnetic field (14) or the strength of the electric DC voltage and AC voltage (Ui, Uij) and can be controlled thereby.

12. Use of the carrier material (11) according to any one of claims 2, 3 or 6 for the translational or rotational movement of electrically polarizable biomaterials, polyelectrolyte materials, atoms, ions and/or molecules (epAIMP) on or at the surface of the carrier material (TM) or between adjacent support material cells (i,j) according to claims 7 to 9, in which the direction of the near-surface electrostatic forces (Fij) is preferably the same direction or preferably opposite direction, **characterized in that** the strength and direction of the near-surface, electrostatic forces (Fij)
a. by applying or changing the applied direct voltage and/or alternating voltage (Ui, Uij) to the rear electrode (5) of individual carrier material cells,
or
b. by irradiation with electromagnetic waves (22), preferably with light whose energy is greater than the band gap (Eg) of the carrier material (TM) or of the or individual carrier material cells (i,j), is changed.

13. Use of the carrier material (11) according to one of claims 6 to 9 for the production of molecular machines (mM), **characterized in that** electrically polarizable biomaterials, atoms, ions or molecules are attached to individual carrier material cells TMZ(i,j) of the carrier material (11) and are moved individually, i.e. separately in succession in time, or completely or partially simultaneously on or in the vicinity of the surface of the carrier material with array structure from a carrier material cell TMZ(i,j) to an adjacent carrier material cell TMZ(i-1,j), TMZ(i+1,j), TMZ(i,j-1) or TMZ(i, j+1), wherein the type of movement is determined by the near-surface electrostatic forces Fij of the carrier material cells TMZ(i,j), **in that** a translational movement of the epAIMP is determined by an overlap region (12) of the near-surface electrostatic forces between adjacent carrier material cells TMZ (i, j) of the array with the same direction of the electrostatic forces and a rotational movement of the epAIMP is determined by an overlap region (12') of the near-surface electrostatic forces between adjacent carrier material cells TMZ (i,j) of the array with different directions of the electrostatic forces

14. Use of the support material (11) according to any one of claims 1 to 9 in an in situ sensor for electrically polarizable biomaterials, polyelectrolyte materials, atoms, ions and/or molecules (epAIMP) in liquids and gases, wherein the sensor surface flow past by the liquids or gases with the epAIMP to be detected is formed by the support material or the support material with array structure.

15. Use of the carrier material (11) according to one of claims 1 to 9 as a filter for electrically polarizable atoms, ions and/or molecules in gases, liquids and powders, wherein the carrier material cells TMZ(i,j) are designed in a screen structure and the filter properties of individual carrier material cells TMZ(i,j) can be controlled by the variable electrostatic forces F(i,j) using claims 10 to 12.

## Revendications

1. Matériau support permettant la modification, la manipulation et le déplacement de biomatériaux, de matériaux polyélectrolytiques, d'atomes, d'ions et/ou de molécules polarisables électriquement (epAIMP), comprenant des matériaux semi-conducteurs ou piézoélectriques, ainsi qu'une couche de revêtement de surface isolante sur la surface des matériaux semi-conducteurs ou piézoélectriques, **caractérisé en ce que** les forces électrostatiques agissent sur l'epAIMP à travers la couche de revêtement de surface, les forces électrostatiques étant générées dans une plage allant jusqu'à 100 nm et leur intensité étant déterminée :
a. par le dipôle électrostatique asymétrique résultant de l'occupation des états d'interface entre le matériau semi-conducteur et la couche de revêtement de surface, la constante de temps des états d'interface (Tᵢⱼ) se situant dans la plage de quelques nanosecondes à des secondes ou la densité d'état d'interface (Dᵢⱼ) variant de 10⁷ à 10¹⁴ cm⁻² eV⁻¹ varie, ainsi que du dopage proche de la surface du matériau semi-conducteur avec des densités de charges volumiques de 10¹⁵ à 10²¹ e/cm³ et des densités de charges de surface de 10¹⁰ à 10¹⁴ e/cm², la densité de charge de surface dans le matériau piézoélectrique étant de 10¹³ à 10¹⁵ e/cm², ou
b. par le nombre de charges de polarisation proches de la surface par unité de surface et l'épaisseur du matériau piézoélectrique
et l'intensité des forces électrostatiques étant toujours déterminée par le type et l'épaisseur du matériau de la couche de revêtement de surface et les forces électrostatiques proches de la surface du matériau support étant indépendantes de l'environnement et les forces électrostatiques proches de la surface ne modifiant pas les autres propriétés de surface du matériau support et l'épaisseur de la couche de revêtement isolante (1) étant inférieure à 20 nm, de préférence inférieure à 5 nm et étant comprise de manière particulièrement préférée entre 2 et 3 nm.

2. Matériau support selon la revendication 1, **caractérisé en ce qu'**au moins une électrode arrière (5) conductrice de métal est disposée sur le côté du matériau support opposé à la couche de revêtement de surface.

3. Matériau support selon la revendication 2, **caractérisé en ce que** l'électrode arrière est structurée.

4. Matériau support selon la revendication 1, **caractérisé en ce que** des impuretés magnétisables (QM) sont introduites dans le matériau support, dans la couche de revêtement isolante (1) ou dans la zone des matériaux semi-conducteurs ou piézoélectriques proches de la surface.

5. Matériau support selon la revendication 1, **caractérisé en ce que** le matériau support
a. comprend un matériau semi-conducteur et que des impuretés chargées individuelles (Q+, Q-, Q+ij, Q-ij) sont introduites localement dans le semi-conducteur,
ou
b. comprend un matériau piézoélectrique et que des impuretés chargées (Q⁺, Q-, Q+ij, Q-ij) sont introduites dans le matériau piézoélectrique,
afin d'influencer les forces électrostatiques (Fij) proches de la surface.

6. Matériau support selon la revendication 2, **caractérisé en ce qu'**au moins une zone de transition est formée entre deux régions adjacentes, avec respectivement la direction inverse des forces électrostatiques proches de la surface dans le matériau support, afin de concevoir de façon modifiable dans le temps et l'espace la manipulation, la modification et le déplacement, par l'application d'une tension à une ou plusieurs électrodes arrières du matériau support, de biomatériaux, de matériaux polyélectrolytiques, d'atomes, d'ions et/ou de molécules polarisables électriquement (epAIMP).

7. Matériau support selon la revendication 1, disposé sous forme de structure en réseau (11), **caractérisé en ce que** le matériau support présente des cellules de matériau support individuelles (i,j) formées à plat par différents polygones, les points d'angle des polygones étant reliés les uns aux autres par paires de manière rectiligne et/ou courbe.

8. Matériau support selon la revendication 7, **caractérisé en ce que** chaque cellule de matériau support (i,j) ou certaines d'entre elles sont équipées de leur propre électrode arrière (5) conductrice de métal.

9. Matériau support selon la revendication 7, **caractérisé en ce que** des impuretés magnétisables (QM) sont introduites dans des cellules de matériau support individuelles, dans la couche de revêtement isolante (1) ou dans la zone proche de la surface des matériaux semi-conducteurs ou piézoélectriques.

10. Utilisation du matériau support selon l'une des revendications 2, 3 ou 6 pour la manipulation de biomatériaux, de matériaux polyélectrolytiques, d'atomes, d'ions et/ou de molécules polarisables électriquement (epAIMP) sur la surface du matériau support (TM) ou entre des cellules de matériau support (TMZ) adjacentes selon l'une des revendications 7 à 9, **caractérisée en ce**
a. **qu'**une tension (U, Ui, Uij, UK) est appliquée ou modifiée sur au moins une des électrodes arrières (5) du matériau support (TM) ou
des cellules de matériau support (TMZ),
ou
b. **que** le matériau support est irradié avec une onde électromagnétique (22) constante ou variable dans le temps avec une photoénergie supérieure à la bande interdite du matériau semi-conducteur, du matériau piézoélectrique ou du matériau de la couche de revêtement isolante,
de sorte que les supports de charges libres dans le matériau semi-conducteur ou les charges de polarisation dans le matériau piézoélectrique sont redistribués et que l'intensité et/ou la direction des forces électrostatiques F(i,j) proches de la surface de la cellule de matériau support TMZ(i,j) sont modifiées.

11. Utilisation du matériau support selon l'une des revendications 2, 3 ou 6 ou de cellules de matériau support selon l'une des revendications 7 à 9 pour la modification de biomatériaux, de matériaux polyélectrolytiques, d'atomes, d'ions et/ou de molécules polarisables électriquement (epAIMP), **caractérisée en ce que**
a. des cellules de matériau support (i,j) individuelles ou le matériau support (TM) sont irradiés avec des ondes électromagnétiques (22), de préférence avec de la lumière, dont l'énergie est supérieure à la bande interdite du matériau support ou de la ou des cellules de matériau support individuelles
ou
b. un champ magnétique externe (14) est appliqué pour inverser l'aimantation des impuretés magnétisables (QM), de préférence un champ magnétique externe alternatif,
ou
c. une tension continue et/ou une tension alternative électriques (Ui, Uij) permettant de charger et de décharger les pièges de charge/impuretés chargés (Q⁺, Q-, Q+ij, Q-ij) sont appliquées à l'électrode arrière (5),
de sorte que l'intensité de la modification des propriétés chimiques et/ou physiques des epAIMP au niveau des cellules de matériau support (i,j) individuelles ou du matériau support dépend de l'amplitude des ondes électromagnétiques (22), de l'intensité du champ magnétique externe (14) ou de l'intensité de la tension continue et de la tension alternative électriques (Ui, Uij) et peut ainsi être contrôlée.

12. Utilisation du matériau support (11) selon l'une des revendications 2, 3 ou 6 pour le déplacement de translation ou de rotation de biomatériaux, de matériaux polyélectrolytiques, d'atomes, d'ions et/ou de molécules polarisables électriquement (epAIMP) sur ou à la surface du matériau support (TM) ou entre des cellules de matériau support (i,j) adjacentes selon les revendications 7 à 9, dans lesquelles la direction des forces électrostatiques (Fij) proches de la surface est de préférence dirigée dans la même direction ou de préférence dans la direction opposée, **caractérisée en ce que** l'intensité et la direction des forces électrostatiques (Fij) proches de la surface sont modifiées
a. en appliquant ou en modifiant la tension continue et/ou la tension alternative (Ui, Uij) appliquées à l'électrode arrière (5) de cellules de matériau support individuelles,
ou
b. en irradiant avec des ondes électromagnétiques (22), de préférence avec de la lumière, dont l'énergie est supérieure à la bande interdite (Eg) du matériau support (TM) ou de la ou des cellules de matériau support (i,j) individuelles.

13. Utilisation du matériau support (11) selon l'une des revendications 6 à 9 pour la fabrication de machines moléculaires (mM), **caractérisée en ce que** des biomatériaux, des atomes, des ions ou des molécules polarisables électriquement se trouvent sur des cellules de matériau support TMZ(i,j) individuelles du matériau support (11) et peuvent être déplacés individuellement, c'est-à-dire séparément dans le temps, ou complètement ou partiellement simultanément sur ou à proximité de la surface du matériau support avec une structure en réseau d'une cellule de matériau support TMZ(i,j) à une cellule de matériau support adjacente TMZ(i-1,j), TMZ(i+1,j), TMZ(i,j-1) ou TMZ(i, j+1), le type de déplacement étant déterminé par les forces électrostatiques (Fij) proches de la surface des cellules de matériau support TMZ(i,j) par un déplacement de translation des epAIMP à travers une zone de chevauchement (12) des forces électrostatiques proches de la surface entre des cellules de matériau support TMZ(i,j) adjacentes du réseau dans la même direction des forces électrostatiques et par un déplacement de rotation des epAIMP à travers une zone de chevauchement (12') des forces électrostatiques proches de la surface entre des cellules de matériau support TMZ(i,j) adjacentes du réseau dans différentes directions des forces électrostatiques.

14. Utilisation du matériau support (11) selon l'une des revendications 1 à 9 dans un capteur in situ destiné à des biomatériaux, des matériaux polyélectrolytiques, des atomes, des ions et/ou des molécules polarisables électriquement (epAIMP) dans des liquides et des gaz, la surface de capteur, sur laquelle s'écoulent les liquides ou les gaz comportant les epAIMP à détecter, étant formée par le matériau support ou le matériau support comportant la structure en réseau.

15. Utilisation du matériau support (11) selon l'une des revendications 1 à 9 comme filtre pour atomes, ions et/ou molécules polarisables électriquement dans des gaz, liquides et poudres, les cellules de matériau support TMZ(i,j) étant conçues dans une structure de tamis et les propriétés de filtrage des cellules de matériau support TMZ(i,j) individuelles pouvant être contrôlées grâce aux forces électrostatiques variables F(i,j) utilisées selon les revendications 10 à 12.
